# EUROPEAN PATENT SPECIFICATION

(11) **EP 1 841 420 B1**
(45) Date of publication and mention of the grant of the patent: **07.12.2011**
(21) Application number: 06717284.1
(22) Date of filing: 04.01.2006
(51) Int. Cl.: A01N 41/10, A01N 43/06, A01N 43/08, A61K 31/10, A61K 31/34, A61K 31/38, C07C 315/00, C07C 317/00

(54) **TREATMENT OF DRUG-RESISTANT PROLIFERATIVE DISORDERS**
BEHANDLUNG HEILMITTELRESISTENTER PROLIFERATIVER ERKRANKUNGEN
TRAITEMENT DE MALADIES PROLIFERANTES PHARMACORESISTANTES

(30) Priority: 05.01.2005 US 641378 P
(43) Date of publication of application: 10.10.2007
(73) Proprietor: Temple University - Of The Commonwealth System of Higher Education, Philadelphia, PA 19122 (US)
(72) Inventor: REDDY, M.V. Ramana, Upper Darby, Pennsylvania 19802-5429 (US); REDDY, E. Premkumar,, Villanova, Pennsylvania 19085 (US); COSENZA, Stephen, C., Voorhees, New Jersey 08043 (US); BAKER, Stacey, J., Philadelphia, Pennsylvania 19116 (US)
(74) Representative: Ouzman, Beverley Nicola Claire
(86) International application number: PCT/US2006/000059
(87) International publication number: WO 2006/074149

(56) References cited:
- WO-A-03/072062
- WO-A-03/072063
- US-B1- 6 599 932
- US-B1- 6 762 207
- US-B2- 6 486 210
- US-B2- 6 548 553
- GUMIREDDY KIRANMAI ET AL: "A non-ATP-competitive inhibitor of BCR-ABL overrides imatinib resistance" PROCEEDINGS OF THE NATIONAL ACADEMY OF SCIENCES OF USA, NATIONAL ACADEMY OF SCIENCE, WASHINGTON, DC, US, vol. 102, no. 6, 8 February 2005 (2005-02-08), pages 1992-1997, XP002405719 ISSN: 0027-8424
- SHAH NEIL P ET AL: "Overriding imatinib resistance with a novel ABL kinase inhibitor." SCIENCE (NEW YORK, N.Y.) 16 JUL 2004, vol. 305, no. 5682, 16 July 2004 (2004-07-16), pages 399-401, XP002552404 ISSN: 1095-9203
- DAUB HENRIK ET AL: "Strategies to overcome resistance to targeted protein kinase inhibitors." NATURE REVIEWS. DRUG DISCOVERY DEC 2004, vol. 3, no. 12, December 2004 (2004-12), pages 1001-1010, XP002552405 ISSN: 1474-1776
- '"Chemotherapeutic Regimens". Drug Facts and Comparisons', vol. EDITION, 1994 pages 2596 - 2604
- NARDI V. ET AL.: 'Mechanisms and Implications of Imatinib Resistance Mutations in BCR-ABL' CURRENT OPINION IN HEMATOLOGY vol. 11, 2004, pages 35 - 43, XP009124799
- CARTER T.A. ET AL.: 'Inhibition of Drug-Resistant Mutants of ABL, KIT and EGF Receptor Kinases' PROCEEDINGS OF THE NATIONAL ACADEMY OF THE SCIENCES OF THE UNITED STATES OF AMERICA vol. 102, no. 31, August 2005, pages 11011 - 11016, XP002440841
- BLACKLEDGE G. ET AL.: 'Gefitinib ('Iressa', ZD 1839) and New Epidermal Growth Factor Receptor Inhibitors' BRITISH JOURNAL OF CANCER vol. 90, 2004, pages 566 - 572, XP008121226
- ZHANG P. ET AL.: 'Gileevec (STI-571 Inhibits Lung Cancer Cell Growth (A549) and Potentiates the Cisplatin Effects In Vitro' MOLECULAR CANCER vol. 2, no. 1, January 2003, pages 1 - 9, XP021018247

### SEQUENCE LISTING

<110> Temple university-of the Commonwealth system of Higher Education
   M.V. Ramana Reddy
   E. Premkumar Reddy
   Stephen C. Cosenza
   Stacey J. Baker
<120> Method of Treating Drug-Resistant
   Proliferative Disorders
<130> 6056-347PC1
<140> unknown
   <141>
<150> US 60/641,378
   <151> 2005-01-05
<160> 5
<170> FastSEQ for windows version 4.0
<210> 1
   <211> 1130
   <212> PRT
   <213> Homo sapiens
<400> 1
   Met Leu Glu Ile Cys Leu Lys Leu Val Gly Cys Lys Ser Lys Lys Gly
   1               5                   10                  15
   Leu Ser Ser Ser Ser Ser Cys Tyr Leu Glu Glu Ala Leu Gln Arg Pro
               20                  25                  30
   Val Ala Ser Asp Phe Glu Pro Gln Gly Leu Ser Glu Ala Ala Arg Trp
           35                  40                  45
   Asn Ser Lys Glu Asn Leu Leu Ala Gly Pro Ser Glu Asn Asp Pro Asn
       50                  55                  60
   Leu Phe Val Ala Leu Tyr Asp Phe Val Ala Ser Gly Asp Asn Thr Leu
   65                  70                  75                  80
   Ser Ile Thr Lys Gly Glu Lys Leu Arg Val Leu Gly Tyr Asn His Asn
                   85                  90                  95
   Gly Glu Trp Cys Glu Ala Gln Thr Lys Asn Gly Gln Gly Trp Val Pro
               100                 105                 110
   Ser Asn Tyr Ile Thr Pro Val Asn Ser Leu Glu Lys His Ser Trp Tyr
           115                 120                 125
   His Gly Pro Val Ser Arg Asn Ala Ala Glu Tyr Leu Leu Ser Ser Gly
       130                 135                 140
   Ile Asn Gly Ser Phe Leu Val Arg Glu Ser Glu Ser Ser Pro Gly Gln
   145                 150                 155                 160
   Arg Ser Ile Ser Leu Arg Tyr Glu Gly Arg Val Tyr His Tyr Arg Ile
                   165                 170                 175
   Asn Thr Ala Ser Asp Gly Lys Leu Tyr Val Ser Ser Glu Ser Arg Phe
               180                 185                 190
   Asn Thr Leu Ala Glu Leu Val His His His Ser Thr Val Ala Asp Gly
           195                 200                 205
   Leu Ile Thr Thr Leu His Tyr Pro Ala Pro Lys Arg Asn Lys Pro Thr
       210                 215                 220
   Val Tyr Gly Val Ser Pro Asn Tyr Asp Lys Trp Glu Met Glu Arg Thr
   225                 230                 235                 240
   Asp Ile Thr Met Lys His Lys Leu Gly Gly Gly Gln Tyr Gly Glu Val
                   245                 250                 255
   Tyr Glu Gly Val Trp Lys Lys Tyr Ser Leu Thr Val Ala Val Lys Thr
               260                 265                 270
   Leu Lys Glu Asp Thr Met Glu Val Glu Glu Phe Leu Lys Glu Ala Ala
           275                 280                 285
   Val Met Lys Glu Ile Lys His Pro Asn Leu Val Gln Leu Leu Gly Val
       290                 295                 300
   Cys Thr Arg Glu Pro Pro Phe Tyr Ile Ile Thr Glu Phe Met Thr Tyr
   305                 310                 315                 320
   Gly Asn Leu Leu Asp Tyr Leu Arg Glu Cys Asn Arg Gln Glu Val Asn
                   325                 330                 335
   Ala Val Val Leu Leu Tyr Met Ala Thr Gln Ile Ser Ser Ala Met Glu
               340                 345                 350
   Tyr Leu Glu Lys Lys Asn Phe Ile His Arg Asp Leu Ala Ala Arg Asn
           355                 360                 365
   Cys Leu Val Gly Glu Asn His Leu Val Lys Val Ala Asp Phe Gly Leu
       370                 375                 380
   Ser Arg Leu Met Thr Gly Asp Thr Tyr Thr Ala His Ala Gly Ala Lys
   385                 390                 395                 400
   Phe Pro Ile Lys Trp Thr Ala Pro Glu Ser Leu Ala Tyr Asn Lys Phe
                   405                 410                 415
   Ser Ile Lys Ser Asp Val Trp Ala Phe Gly Val Leu Leu Trp Glu Ile
               420                 425                 430
   Ala Thr Tyr Gly Met Ser Pro Tyr Pro Gly Ile Asp Leu Ser Gln Val
           435                 440                 445
   Tyr Glu Leu Leu Glu Lys Asp Tyr Arg Met Glu Arg Pro Glu Gly Cys
       450                 455                 460
   Pro Glu Lys Val Tyr Glu Leu Met Arg Ala Cys Trp Gln Trp Asn Pro
   465                 470                 475                 480
   Ser Asp Arg Pro Ser Phe Ala Glu Ile His Gln Ala Phe Glu Thr Met
                   485                 490                 495
   Phe Gln Glu Ser Ser Ile Ser Asp Glu Val Glu Lys Glu Leu Gly Lys
               500                 505                 510
   Gln Gly Val Arg Gly Ala Val Ser Thr Leu Leu Gln Ala Pro Glu Leu
           515                 520                 525
   Pro Thr Lys Thr Arg Thr Ser Arg Arg Ala Ala Glu His Arg Asp Thr
       530                 535                 540
   Thr Asp Val Pro Glu Met Pro His Ser Lys Gly Gln Gly Glu Ser Asp
   545                 550                 555                 560
   Pro Leu Asp His Glu Pro Ala Val Ser Pro Leu Leu Pro Arg Lys Glu
                   565                 570                 575
   Arg Gly Pro Pro Glu Gly Gly Leu Asn Glu Asp Glu Arg Leu Leu Pro
               580                 585                 590
   Lys Asp Lys Lys Thr Asn Leu Phe Ser Ala Leu Ile Lys Lys Lys Lys
           595                 600                 605
   Lys Thr Ala Pro Thr Pro Pro Lys Arg Ser Ser Ser Phe Arg Glu Met
       610                 615                 620
   Asp Gly Gln Pro Glu Arg Arg Gly Ala Gly Glu Glu Glu Gly Arg Asp
   625                 630                 635                 640
   Ile Ser Asn Gly Ala Leu Ala Phe Thr Pro Leu Asp Thr Ala Asp Pro
                   645                 650                 655
   Ala Lys Ser Pro Lys Pro Ser Asn Gly Ala Gly Val Pro Asn Gly Ala
               660                 665                 670
   Leu Arg Glu Ser Gly Gly Ser Gly Phe Arg Ser Pro His Leu Trp Lys
           675                 680                 685
   Lys Ser Ser Thr Leu Thr Ser Ser Arg Leu Ala Thr Gly Glu Glu Glu
       690                 695                 700
   Gly Gly Gly Ser Ser Ser Lys Arg Phe Leu Arg Ser Cys Ser Ala Ser
   705                 710                 715                 720
   Cys Val Pro His Gly Ala Lys Asp Thr Glu Trp Arg Ser Val Thr Leu
                   725                 730                 735
   Pro Arg Asp Leu Gln Ser Thr Gly Arg Gln Phe Asp Ser Ser Thr Phe
               740                 745                 750
   Gly Gly His Lys Ser Glu Lys Pro Ala Leu Pro Arg Lys Arg Ala Gly
           755                 760                 765
   Glu Asn Arg Ser Asp Gln Val Thr Arg Gly Thr Val Thr Pro Pro Pro
       770                 775                 780
   Arg Leu Val Lys Lys Asn Glu Glu Ala Ala Asp Glu Val Phe Lys Asp
   785                 790                 795                 800
   Ile Met Glu Ser Ser Pro Gly Ser Ser Pro Pro Asn Leu Thr Pro Lys
                   805                 810                 815
   Pro Leu Arg Arg Gln Val Thr Val Ala Pro Ala Ser Gly Leu Pro His
               820                 825                 830
   Lys Glu Glu Ala Gly Lys Gly Ser Ala Leu Gly Thr Pro Ala Ala Ala
   835 840 845
   Glu Pro Val Thr Pro Thr Ser Lys Ala Gly Ser Gly Ala Pro Gly Gly
   850 855 860
   Thr Ser Lys Gly Pro Ala Glu Glu Ser Arg Val Arg Arg His Lys His
   865 870 875 880
   Ser Ser Glu Ser Pro Gly Arg Asp Lys Gly Lys Leu Ser Arg Leu Lys
   885 890 895
   Pro Ala Pro Pro Pro Pro Pro Ala Ala Ser Ala Gly Lys Ala Gly Gly
   900 905 910
   Lys Pro Ser Gln Ser Pro Ser Gln Glu Ala Ala Gly Glu Ala Val Leu
   915 920 925
   Gly Ala Lys Thr Lys Ala Thr Ser Leu Val Asp Ala Val Asn Ser Asp
   930 935 940
   Ala Ala Lys Pro Ser Gln Pro Gly Glu Gly Leu Lys Lys Pro Val Leu
   945 950 955 960
   Pro Ala Thr Pro Lys Pro Gln Ser Ala Lys Pro Ser Gly Thr Pro Ile
   965 970 975
   Ser Pro Ala Pro Val Pro Ser Thr Leu Pro Ser Ala Ser Ser Ala Leu
   980 985 990
   Ala Gly Asp Gln Pro Ser Ser Thr Ala Phe Ile Pro Leu Ile Ser Thr
   995 1000 1005
   Arg Val Ser Leu Arg Lys Thr Arg Gln Pro Pro Glu Arg Ile Ala Ser
   1010 1015 1020
   Gly Ala Ile Thr Lys Gly Val Val Leu Asp Ser Thr Glu Ala Leu Cys
   1025 1030 1035 1040
   Leu Ala Ile Ser Arg Asn Ser Glu Gln Met Ala Ser His Ser Ala Val
   1045 1050 1055
   Leu Glu Ala Gly Lys Asn Leu Tyr Thr Phe Cys Val Ser Tyr Val Asp
   1060 1065 1070
   Ser Ile Gln Gln Met Arg Asn Lys Phe Ala Phe Arg Glu Ala Ile Asn
   1075 1080 1085
   Lys Leu Glu Asn Asn Leu Arg Glu Leu Gln Ile Cys Pro Ala Thr Ala
   1090 1095 1100
   Gly Ser Gly Pro Ala Ala Thr Gln Asp Phe Ser Lys Leu Leu Ser Ser
   1105 1110 1115 1120
   Val Lys Glu Ile Ser Asp Ile Val Gln Arg
   1125 1130
<210> 2
   <211> 1089
   <212> PRT
   <213> Homo sapiens
<400> 2
<210> 3
   <211> 1106
   <212> PRT
   <213> Homo sapiens
<400> 3
<210> 4
   <211> 976
   <212> PRT
   <213> Homo sapiens
<400> 4
<210> 5
   <211> 1186
   <212> PRT
   <213> Homo sapiens
<400> 5
   Leu Glu Glu Lys Lys Val Cys Gln Gly Thr Ser Asn Lys Leu Thr Gln
   1               5                   10                  15
   Leu Gly Thr Phe Glu Asp His Phe Leu Ser Leu Gln Arg Met Phe Asn
               20                  25                  30
   Asn Cys Glu Val Val Leu Gly Asn Leu Glu Ile Thr Tyr Val Gln Arg
           35                  40                  45
   Asn Tyr Asp Leu Ser Phe Leu Lys Thr Ile Gln Glu Val Ala Gly Tyr
   50 55 60
   Val Leu Ile Ala Leu Asn Thr Val Glu Arg Ile Pro Leu Glu Asn Leu
   65 70 75 80
   Gln Ile Ile Arg Gly Asn Met Tyr Tyr Glu Asn Ser Tyr Ala Leu Ala
   85 90 95
   Val Leu Ser Asn Tyr Asp Ala Asn Lys Thr Gly Leu Lys Glu Leu Pro
   100 105 110
   Met Arg Asn Leu Gln Glu Ile Leu His Gly Ala Val Arg Phe Ser Asn
   115 120 125
   Asn Pro Ala Leu Cys Asn Val Glu Ser Ile Gln Trp Arg Asp Ile Val
   130 135 140
   Ser Ser Asp Phe Leu Ser Asn Met Ser Met Asp Phe Gln Asn His Leu
   145 150 155 160
   Gly Ser Cys Gln Lys Cys Asp Pro Ser Cys Pro Asn Gly Ser Cys Trp
   165 170 175
   Gly Ala Gly Glu Glu Asn Cys Gln Lys Leu Thr Lys Ile Ile Cys Ala
   180 185 190
   Gln Gln Cys Ser Gly Arg Cys Arg Gly Lys Ser Pro Ser Asp Cys Cys
   195 200 205
   His Asn Gln Cys Ala Ala Gly Cys Thr Gly Pro Arg Glu Ser Asp Cys
   210 215 220
   Leu Val Cys Arg Lys Phe Arg Asp Glu Ala Thr Cys Lys Asp Thr Cys
   225 230 235 240
   Pro Pro Leu Met Leu Tyr Asn Pro Thr Thr Tyr Gln Met Asp Val Asn
   245 250 255
   Pro Glu Gly Lys Tyr Ser Phe Gly Ala Thr Cys Val Lys Lys Cys Pro
   260 265 270
   Arg Asn Tyr Val Val Thr Asp His Gly Ser Cys Val Arg Ala Cys Gly
   275 280 285
   Ala Asp Ser Tyr Glu Met Glu Glu Asp Gly Val Arg Lys Cys Lys Lys
   290 295 300
   Cys Glu Gly Pro Cys Arg Lys Val Cys Asn Gly Ile Gly Ile Gly Glu
   305 310 315 320
   Phe Lys Asp Ser Leu Ser Ile Asn Ala Thr Asn Ile Lys His Phe Lys
   325 330 335
   Asn Cys Thr Ser Ile Ser Gly Asp Leu His Ile Leu Pro Val Ala Phe
   340 345 350
   Arg Gly Asp Ser Phe Thr His Thr Pro Pro Leu Asp Pro Gln Glu Leu
   355 360 365
   Asp Ile Leu Lys Thr Val Lys Glu Ile Thr Gly Phe Leu Leu Ile Gln
   370 375 380
   Ala Trp Pro Glu Asn Arg Thr Asp Leu His Ala Phe Glu Asn Leu Glu
   385 390 395 400
   Ile Ile Arg Gly Arg Thr Lys Gln His Gly Gln Phe Ser Leu Ala Val
   405 410 415
   Val Ser Leu Asn Ile Thr Ser Leu Gly Leu Arg Ser Leu Lys Glu Ile
   420 425 430
   Ser Asp Gly Asp Val Ile Ile Ser Gly Asn Lys Asn Leu Cys Tyr Ala
   435 440 445
   Asn Thr Ile Asn Trp Lys Lys Leu Phe Gly Thr Ser Gly Gln Lys Thr
   450 455 460
   Lys Ile Ile Ser Asn Arg Gly Glu Asn Ser Cys Lys Ala Thr Gly Gln
   465 470 475 480
   Val Cys His Ala Leu Cys Ser Pro Glu Gly Cys Trp Gly Pro Glu Pro
   485 490 495
   Arg Asp Cys Val Ser Cys Arg Asn Val Ser Arg Gly Arg Glu Cys Val
   500 505 510
   Asp Lys Cys Lys Leu Leu Glu Gly Glu Pro Arg Glu Phe Val Glu Asn
   515 520 525
   Ser Glu Cys Ile Gln Cys His Pro Glu Cys Leu Pro Gln Ala Met Asn
   530 535 540
   Ile Thr Cys Thr Gly Arg Gly Pro Asp Asn Cys Ile Gln Cys Ala His
   545 550 555 560
   Tyr Ile Asp Gly Pro His Cys Val Lys Thr Cys Pro Ala Gly Val Met
   565 570 575
   Gly Glu Asn Asn Thr Leu Val Trp Lys Tyr Ala Asp Ala Gly His Val
   580 585 590
   Cys His Leu Cys His Pro Asn Cys Thr Tyr Gly Cys Thr Gly Pro Gly
           595                 600                 605
   Leu Glu Gly Cys Pro Thr Asn Gly Pro Lys Ile Pro Ser Ile Ala Thr
       610                 615                 620
   Gly Met Val Gly Ala Leu Leu Leu Leu Leu Val Val Ala Leu Gly Ile
   625                 630                 635                 640
   Gly Leu Phe Met Arg Arg Arg His Ile Val Arg Lys Arg Thr Leu Arg
                   645                 650                 655
   Arg Leu Leu Gln Glu Arg Glu Leu Val Glu Pro Leu Thr Pro Ser Gly
               660                 665                 670
   Glu Ala Pro Asn Gln Ala Leu Leu Arg Ile Leu Lys Glu Thr Glu Phe
           675                 680                 685
   Lys Lys Ile Lys Val Leu Gly Ser Gly Ala Phe Gly Thr Val Tyr Lys
       690                 695                 700
   Gly Leu Trp Ile Pro Glu Gly Glu Lys Val Lys Ile Pro Val Ala Ile
   705                 710                 715                 720
   Lys Glu Leu Arg Glu Ala Thr Ser Pro Lys Ala Asn Lys Glu Ile Leu
                   725                 730                 735
   Asp Glu Ala Tyr Val Met Ala Ser Val Asp Asn Pro His Val Cys Arg
               740                 745                 750
   Leu Leu Gly Ile Cys Leu Thr Ser Thr Val Gln Leu Ile Thr Gln Leu
           755                 760                 765
   Met Pro Phe Gly Cys Leu Leu Asp Tyr Val Arg Glu His Lys Asp Asn
       770                 775                 780
   Ile Gly Ser Gln Tyr Leu Leu Asn Trp Cys Val Gln Ile Ala Lys Gly
   785                 790                 795                 800
   Met Asn Tyr Leu Glu Asp Arg Arg Leu Val His Arg Asp Leu Ala Ala
                   805                 810                 815
   Arg Asn Val Leu Val Lys Thr Pro Gln His Val Lys Ile Thr Asp Phe
               820                 825                 830
   Gly Leu Ala Lys Leu Leu Gly Ala Glu Glu Lys Glu Tyr His Ala Glu
           835                 840                 845
   Gly Gly Lys Val Pro Ile Lys Trp Met Ala Leu Glu Ser Ile Leu His
       850                 855                 860
   Arg Ile Tyr Thr His Gln Ser Asp Val Trp Ser Tyr Gly Val Thr Val
   865                 870                 875                 880
   Trp Glu Leu Met Thr Phe Gly Ser Lys Pro Tyr Asp Gly Ile Pro Ala
                   885                 890                 895
   Ser Glu Ile Ser Ser Ile Leu Glu Lys Gly Glu Arg Leu Pro Gln Pro
               900                 905                 910
   Pro Ile Cys Thr Ile Asp Val Tyr Met Ile Met Val Lys Cys Trp Met
           915                 920                 925
   Ile Asp Ala Asp Ser Arg Pro Lys Phe Arg Glu Leu Ile Ile Glu Phe
       930                 935                 940
   Ser Lys Met Ala Arg Asp Pro Gln Arg Tyr Leu Val Ile Gln Gly Asp
   945                 950                 955                 960
   Glu Arg Met His Leu Pro Ser Pro Thr Asp Ser Asn Phe Tyr Arg Ala
                   965                 970                 975
   Leu Met Asp Glu Glu Asp Met Asp Asp Val Val Asp Ala Asp Glu Tyr
               980                 985                 990
   Leu Ile Pro Gln Gln Gly Phe Phe Ser Ser Pro Ser Thr Ser Arg Thr
           995                 1000                1005
   Pro Leu Leu Ser Ser Leu Ser Ala Thr Ser Asn Asn Ser Thr Val Ala
       1010                1015                1020
   Cys Ile Asp Arg Asn Gly Leu Gln Ser Cys Pro Ile Lys Glu Asp Ser
   1025                1030                1035                1040
   Phe Leu Gln Arg Tyr Ser Ser Asp Pro Thr Gly Ala Leu Thr Glu Asp
                   1045                1050                1055
   Ser Ile Asp Asp Thr Phe Leu Pro Val Pro Glu Tyr Ile Asn Gln Ser
               1060                1065                1070
   Val Pro Lys Arg Pro Ala Gly Ser Val Gln Asn Pro Val Tyr His Asn
           1075                1080                1085
   Gln Pro Leu Asn Pro Ala Pro Ser Arg Asp Pro His Tyr Gln Asp Pro
       1090                1095                1100
   His Ser Thr Ala Val Gly Asn Pro Glu Tyr Leu Asn Thr Val Gln Pro
   1105                1110                1115                1120
   Thr Cys Val Asn Ser Thr Phe Asp Ser Pro Ala His Trp Ala Gln Lys
                   1125                1130                1135
   Gly Ser His Gln Ile Ser Leu Asp Asn Pro Asp Tyr Gln Gln Asp Phe
   1140 1145 1150
   Phe Pro Lys Glu Ala Lys Pro Asn Gly Ile Phe Lys Gly Ser Thr Ala
   1155 1160 1165
   Glu Asn Ala Glu Tyr Leu Arg Val Ala Pro Gln Ser Ser Glu Phe Ile
   1170 1175 1180
   Gly Ala
   1185

### SEQUENCE LISTING

<110> Temple university-of the Commonwealth System of Higher Education
   M.V. Ramana Reddy
   E. Premkumar Reddy
   Stephen C. Cosenza
   Stacey J. Baker
<120> Method of Treating Drug-Resistant
   Proliferative Disorders
<130> 35926-0347-00-WO
<140> unknown
   <141>
<150> US 60/641,378
   <151> 2005-01-05
<160> 5
<170> FastSEQ for windows Version 4.0
<210> 1
   <211> 1130
   <212> PRT
   <213> Homo sapiens
<400> 1
   Met Leu Glu Ile Cys Leu Lys Leu Val Gly Cys Lys Ser Lys Lys Gly
   1               5                   10                  15
   Leu Ser Ser Ser Ser Ser Cys Tyr Leu Glu Glu Ala Leu Gln Arg Pro
               20                  25                  30
   Val Ala Ser Asp Phe Glu Pro Gln Gly Leu Ser Glu Ala Ala Arg Trp
           35                  40                  45
   Asn Ser Lys Glu Asn Leu Leu Ala Gly Pro Ser Glu Asn Asp Pro Asn
       50                  55                  60
   Leu Phe Val Ala Leu Tyr Asp Phe Val Ala Ser Gly Asp Asn Thr Leu
   65                  70                  75                  80
   Ser Ile Thr Lys Gly Glu Lys Leu Arg Val Leu Gly Tyr Asn His Asn
                   85                  90                  95
   Gly Glu Trp Cys Glu Ala Gln Thr Lys Asn Gly Gln Gly Trp Val Pro
               100                 105                 110
   Ser Asn Tyr Ile Thr Pro Val Asn Ser Leu Glu Lys His Ser Trp Tyr
           115                 120                 125
   His Gly Pro Val Ser Arg Asn Ala Ala Glu Tyr Leu Leu Ser Ser Gly
       130                 135                 140
   Ile Asn Gly Ser Phe Leu Val Arg Glu Ser Glu Ser Ser Pro Gly Gln
   145                 150                 155                 160
   Arg Ser Ile Ser Leu Arg Tyr Glu Gly Arg Val Tyr His Tyr Arg Ile
                   165                 170                 175
   Asn Thr Ala Ser Asp Gly Lys Leu Tyr Val Ser Ser Glu Ser Arg Phe
               180                 185                 190
   Asn Thr Leu Ala Glu Leu Val His His His Ser Thr Val Ala Asp Gly
           195                 200                 205
   Leu Ile Thr Thr Leu His Tyr Pro Ala Pro Lys Arg Asn Lys Pro Thr
       210                 215                 220
   Val Tyr Gly Val Ser Pro Asn Tyr Asp Lys Trp Glu Met Glu Arg Thr
   225                 230                 235                 240
   Asp Ile Thr Met Lys His Lys Leu Gly Gly Gly Gln Tyr Gly Glu Val
                   245                 250                 255
   Tyr Glu Gly Val Trp Lys Lys Tyr Ser Leu Thr Val Ala Val Lys Thr
               260                 265                 270
   Leu Lys Glu Asp Thr Met Glu Val Glu Glu Phe Leu Lys Glu Ala Ala
           275                 280                 285
   Val Met Lys Glu Ile Lys His Pro Asn Leu Val Gln Leu Leu Gly Val
       290                 295                 300
   Cys Thr Arg Glu Pro Pro Phe Tyr Ile Ile Thr Glu Phe Met Thr Tyr
   305                 310                 315                 320
   Gly Asn Leu Leu Asp Tyr Leu Arg Glu Cys Asn Arg Gln Glu Val Asn
                   325                 330                 335
   Ala Val Val Leu Leu Tyr Met Ala Thr Gln Ile Ser Ser Ala Met Glu
               340                 345                 350
   Tyr Leu Glu Lys Lys Asn Phe Ile His Arg Asp Leu Ala Ala Arg Asn
           355                 360                 365
   Cys Leu Val Gly Glu Asn His Leu Val Lys Val Ala Asp Phe Gly Leu
       370                 375                 380
   Ser Arg Leu Met Thr Gly Asp Thr Tyr Thr Ala His Ala Gly Ala Lys
   385                 390                 395                 400
   Phe Pro Ile Lys Trp Thr Ala Pro Glu Ser Leu Ala Tyr Asn Lys Phe
                   405                 410                 415
   Ser Ile Lys Ser Asp Val Trp Ala Phe Gly Val Leu Leu Trp Glu Ile
               420                 425                 430
   Ala Thr Tyr Gly Met Ser Pro Tyr Pro Gly Ile Asp Leu Ser Gln Val
           435                 440                 445
   Tyr Glu Leu Leu Glu Lys Asp Tyr Arg Met Glu Arg Pro Glu Gly Cys
       450                 455                 460
   Pro Glu Lys Val Tyr Glu Leu Met Arg Ala Cys Trp Gln Trp Asn Pro
   465                 470                 475                 480
   Ser Asp Arg Pro Ser Phe Ala Glu Ile His Gln Ala Phe Glu Thr Met
                   485                 490                 495
   Phe Gln Glu Ser Ser Ile Ser Asp Glu Val Glu Lys Glu Leu Gly Lys
               500                 505                 510
   Gln Gly Val Arg Gly Ala Val Ser Thr Leu Leu Gln Ala Pro Glu Leu
           515                 520                 525
   Pro Thr Lys Thr Arg Thr Ser Arg Arg Ala Ala Glu His Arg Asp Thr
       530                 535                 540
   Thr Asp Val Pro Glu Met Pro His Ser Lys Gly Gln Gly Glu Ser Asp
   545                 550                 555                 560
   Pro Leu Asp His Glu Pro Ala Val Ser Pro Leu Leu Pro Arg Lys Glu
                   565                 570                 575
   Arg Gly Pro Pro Glu Gly Gly Leu Asn Glu Asp Glu Arg Leu Leu Pro
               580                 585                 590
   Lys Asp Lys Lys Thr Asn Leu Phe Ser Ala Leu Ile Lys Lys Lys Lys
           595                 600                 605
   Lys Thr Ala Pro Thr Pro Pro Lys Arg Ser Ser Ser Phe Arg Glu Met
       610                 615                 620
   Asp Gly Gln Pro Glu Arg Arg Gly Ala Gly Glu Glu Glu Gly Arg Asp
   625                 630                 635                 640
   Ile Ser Asn Gly Ala Leu Ala Phe Thr Pro Leu Asp Thr Ala Asp Pro
                   645                 650                 655
   Ala Lys Ser Pro Lys Pro Ser Asn Gly Ala Gly Val Pro Asn Gly Ala
               660                 665                 670
   Leu Arg Glu Ser Gly Gly Ser Gly Phe Arg Ser Pro His Leu Trp Lys
           675                 680                 685
   Lys Ser Ser Thr Leu Thr Ser Ser Arg Leu Ala Thr Gly Glu Glu Glu
       690                 695                 700
   Gly Gly Gly Ser Ser Ser Lys Arg Phe Leu Arg Ser Cys Ser Ala Ser
   705                 710                 715                 720
   Cys Val Pro His Gly Ala Lys Asp Thr Glu Trp Arg Ser Val Thr Leu
                   725                 730                 735
   Pro Arg Asp Leu Gln Ser Thr Gly Arg Gln Phe Asp Ser Ser Thr Phe
               740                 745                 750
   Gly Gly His Lys Ser Glu Lys Pro Ala Leu Pro Arg Lys Arg Ala Gly
           755                 760                 765
   Glu Asn Arg Ser Asp Gln Val Thr Arg Gly Thr Val Thr Pro Pro Pro
       770                 775                 780
   Arg Leu Val Lys Lys Asn Glu Glu Ala Ala Asp Glu Val Phe Lys Asp
   785                 790                 795                 800
   Ile Met Glu Ser Ser Pro Gly Ser Ser Pro Pro Asn Leu Thr Pro Lys
                   805                 810                 815
   Pro Leu Arg Arg Gln Val Thr Val Ala Pro Ala Ser Gly Leu Pro His
               820                 825                 830
   Lys Glu Glu Ala Gly Lys Gly Ser Ala Leu Gly Thr Pro Ala Ala Ala
           835                 840                 845
   Glu Pro Val Thr Pro Thr Ser Lys Ala Gly Ser Gly Ala Pro Gly Gly
       850                 855                 860
   Thr Ser Lys Gly Pro Ala Glu Glu Ser Arg Val Arg Arg His Lys His
   865                 870                 875                 880
   Ser Ser Glu Ser Pro Gly Arg Asp Lys Gly Lys Leu Ser Arg Leu Lys
                   885                 890                 895
   Pro Ala Pro Pro Pro Pro Pro Ala Ala Ser Ala Gly Lys Ala Gly Gly
               900                 905                 910
   Lys Pro Ser Gln Ser Pro Ser Gln Glu Ala Ala Gly Glu Ala Val Leu
           915                 920                 925
   Gly Ala Lys Thr Lys Ala Thr Ser Leu Val Asp Ala Val Asn Ser Asp
       930                 935                 940
   Ala Ala Lys Pro Ser Gln Pro Gly Glu Gly Leu Lys Lys Pro Val Leu
   945                 950                 955                 960
   Pro Ala Thr Pro Lys Pro Gln Ser Ala Lys Pro Ser Gly Thr Pro Ile
                   965                 970                 975
   Ser Pro Ala Pro Val Pro Ser Thr Leu Pro Ser Ala Ser Ser Ala Leu
               980                 985                 990
   Ala Gly Asp Gln Pro Ser Ser Thr Ala Phe Ile Pro Leu Ile Ser Thr
           995                 1000                1005
   Arg Val Ser Leu Arg Lys Thr Arg Gln Pro Pro Glu Arg Ile Ala Ser
       1010                1015                1020
   Gly Ala Ile Thr Lys Gly Val Val Leu Asp Ser Thr Glu Ala Leu Cys
   1025                1030                1035                1040
   Leu Ala Ile Ser Arg Asn Ser Glu Gln Met Ala Ser His Ser Ala Val
                   1045                1050                1055
   Leu Glu Ala Gly Lys Asn Leu Tyr Thr Phe Cys Val Ser Tyr Val Asp
               1060                1065                1070
   Ser Ile Gln Gln Met Arg Asn Lys Phe Ala Phe Arg Glu Ala Ile Asn
           1075                1080                1085
   Lys Leu Glu Asn Asn Leu Arg Glu Leu Gln Ile Cys Pro Ala Thr Ala
       1090                1095                1100
   Gly Ser Gly Pro Ala Ala Thr Gln Asp Phe Ser Lys Leu Leu Ser Ser
   1105                1110                1115                1120
   Val Lys Glu Ile Ser Asp Ile Val Gln Arg
                   1125                1130
<210> 2
   <211> 1089
   <212> PRT
   <213> Homo sapiens
<400> 2
<210> 3
   <211> 1106
   <212> PRT
   <213> Homo sapiens
<400> 3
<210> 4
   <211> 976
   <212> PRT
   <213> Homo sapiens
<400> 4
<210> 5
   <211> 1186
   <212> PRT
   <213> Homo sapiens
<400> 5
   Leu Glu Glu Lys Lys Val Cys Gln Gly Thr Ser Asn Lys Leu Thr Gln
   1               5                   10                  15
   Leu Gly Thr Phe Glu Asp His Phe Leu Ser Leu Gln Arg Met Phe Asn
               20                  25                  30
   Asn Cys Glu Val Val Leu Gly Asn Leu Glu Ile Thr Tyr Val Gln Arg
           35                  40                  45
   Asn Tyr Asp Leu Ser Phe Leu Lys Thr Ile Gln Glu Val Ala Gly Tyr
       50                  55                  60
   Val Leu Ile Ala Leu Asn Thr Val Glu Arg Ile Pro Leu Glu Asn Leu
   65                  70                  75                  80
   Gln Ile Ile Arg Gly Asn Met Tyr Tyr Glu Asn Ser Tyr Ala Leu Ala
                   85                  90                  95
   Val Leu Ser Asn Tyr Asp Ala Asn Lys Thr Gly Leu Lys Glu Leu Pro
               100                 105                 110
   Met Arg Asn Leu Gln Glu Ile Leu His Gly Ala Val Arg Phe Ser Asn
           115                 120                 125
   Asn Pro Ala Leu Cys Asn Val Glu Ser Ile Gln Trp Arg Asp Ile Val
       130                 135                 140
   Ser Ser Asp Phe Leu Ser Asn Met Ser Met Asp Phe Gln Asn His Leu
   145                 150                 155                 160
   Gly Ser Cys Gln Lys Cys Asp Pro Ser Cys Pro Asn Gly Ser Cys Trp
                   165                 170                 175
   Gly Ala Gly Glu Glu Asn Cys Gln Lys Leu Thr Lys Ile Ile Cys Ala
               180                 185                 190
   Gln Gln Cys Ser Gly Arg Cys Arg Gly Lys Ser Pro Ser Asp Cys Cys
           195                 200                 205
   His Asn Gln Cys Ala Ala Gly Cys Thr Gly Pro Arg Glu Ser Asp Cys
       210                 215                 220
   Leu Val Cys Arg Lys Phe Arg Asp Glu Ala Thr Cys Lys Asp Thr Cys
   225                 230                 235                 240
   Pro Pro Leu Met Leu Tyr Asn Pro Thr Thr Tyr Gln Met Asp Val Asn
                   245                 250                 255
   Pro Glu Gly Lys Tyr Ser Phe Gly Ala Thr Cys Val Lys Lys Cys Pro
               260                 265                 270
   Arg Asn Tyr Val Val Thr Asp His Gly Ser Cys Val Arg Ala Cys Gly
           275                 280                 285
   Ala Asp Ser Tyr Glu Met Glu Glu Asp Gly Val Arg Lys Cys Lys Lys
       290                 295                 300
   Cys Glu Gly Pro Cys Arg Lys Val Cys Asn Gly Ile Gly Ile Gly Glu
   305                 310                 315                 320
   Phe Lys Asp Ser Leu Ser Ile Asn Ala Thr Asn Ile Lys His Phe Lys
                   325                 330                 335
   Asn Cys Thr Ser Ile Ser Gly Asp Leu His Ile Leu Pro Val Ala Phe
               340                 345                 350
   Arg Gly Asp Ser Phe Thr His Thr Pro Pro Leu Asp Pro Gln Glu Leu
           355                 360                 365
   Asp Ile Leu Lys Thr Val Lys Glu Ile Thr Gly Phe Leu Leu Ile Gln
       370                 375                 380
   Ala Trp Pro Glu Asn Arg Thr Asp Leu His Ala Phe Glu Asn Leu Glu
   385                 390                 395                 400
   Ile Ile Arg Gly Arg Thr Lys Gln His Gly Gln Phe Ser Leu Ala Val
                   405                 410                 415
   Val Ser Leu Asn Ile Thr Ser Leu Gly Leu Arg Ser Leu Lys Glu Ile
               420                 425                 430
   Ser Asp Gly Asp Val Ile Ile Ser Gly Asn Lys Asn Leu Cys Tyr Ala
           435                 440                 445
   Asn Thr Ile Asn Trp Lys Lys Leu Phe Gly Thr Ser Gly Gln Lys Thr
       450                 455                 460
   Lys Ile Ile Ser Asn Arg Gly Glu Asn Ser Cys Lys Ala Thr Gly Gln
   465                 470                 475                 480
   Val Cys His Ala Leu Cys Ser Pro Glu Gly Cys Trp Gly Pro Glu Pro
                   485                 490                 495
   Arg Asp Cys Val Ser Cys Arg Asn Val Ser Arg Gly Arg Glu Cys Val
               500                 505                 510
   Asp Lys Cys Lys Leu Leu Glu Gly Glu Pro Arg Glu Phe Val Glu Asn
           515                 520                 525
   Ser Glu Cys Ile Gln Cys His Pro Glu Cys Leu Pro Gln Ala Met Asn
       530                 535                 540
   Ile Thr Cys Thr Gly Arg Gly Pro Asp Asn Cys Ile Gln Cys Ala His
   545                 550                 555                 560
   Tyr Ile Asp Gly Pro His Cys Val Lys Thr Cys Pro Ala Gly Val Met
                   565                 570                 575
   Gly Glu Asn Asn Thr Leu Val Trp Lys Tyr Ala Asp Ala Gly His Val
               580                 585                 590
   Cys His Leu Cys His Pro Asn Cys Thr Tyr Gly Cys Thr Gly Pro Gly
           595                 600                 605
   Leu Glu Gly Cys Pro Thr Asn Gly Pro Lys Ile Pro Ser Ile Ala Thr
       610                 615                 620
   Gly Met Val Gly Ala Leu Leu Leu Leu Leu Val Val Ala Leu Gly Ile
   625                 630                 635                 640
   Gly Leu Phe Met Arg Arg Arg His Ile Val Arg Lys Arg Thr Leu Arg
                   645                 650                 655
   Arg Leu Leu Gln Glu Arg Glu Leu Val Glu Pro Leu Thr Pro Ser Gly
               660                 665                 670
   Glu Ala Pro Asn Gln Ala Leu Leu Arg Ile Leu Lys Glu Thr Glu Phe
           675                 680                 685
   Lys Lys Ile Lys Val Leu Gly Ser Gly Ala Phe Gly Thr Val Tyr Lys
       690                 695                 700
   Gly Leu Trp Ile Pro Glu Gly Glu Lys Val Lys Ile Pro Val Ala Ile
   705                 710                 715                 720
   Lys Glu Leu Arg Glu Ala Thr Ser Pro Lys Ala Asn Lys Glu Ile Leu
                   725                 730                 735
   Asp Glu Ala Tyr Val Met Ala Ser Val Asp Asn Pro His Val Cys Arg
               740                 745                 750
   Leu Leu Gly Ile Cys Leu Thr Ser Thr Val Gln Leu Ile Thr Gln Leu
           755                 760                 765
   Met Pro Phe Gly Cys Leu Leu Asp Tyr Val Arg Glu His Lys Asp Asn
       770                 775                 780
   Ile Gly Ser Gln Tyr Leu Leu Asn Trp Cys Val Gln Ile Ala Lys Gly
   785                 790                 795                 800
   Met Asn Tyr Leu Glu Asp Arg Arg Leu Val His Arg Asp Leu Ala Ala
                   805                 810                 815
   Arg Asn Val Leu Val Lys Thr Pro Gln His Val Lys Ile Thr Asp Phe
               820                 825                 830
   Gly Leu Ala Lys Leu Leu Gly Ala Glu Glu Lys Glu Tyr His Ala Glu
           835                 840                 845
   Gly Gly Lys Val Pro Ile Lys Trp Met Ala Leu Glu Ser Ile Leu His
       850                 855                 860
   Arg Ile Tyr Thr His Gln Ser Asp Val Trp Ser Tyr Gly Val Thr Val
   865                 870                 875                 880
   Trp Glu Leu Met Thr Phe Gly Ser Lys Pro Tyr Asp Gly Ile Pro Ala
                   885                 890                 895
   Ser Glu Ile Ser Ser Ile Leu Glu Lys Gly Glu Arg Leu Pro Gln Pro
               900                 905                 910
   Pro Ile Cys Thr Ile Asp Val Tyr Met Ile Met Val Lys Cys Trp Met
           915                 920                 925
   Ile Asp Ala Asp Ser Arg Pro Lys Phe Arg Glu Leu Ile Ile Glu Phe
       930                 935                 940
   Ser Lys Met Ala Arg Asp Pro Gln Arg Tyr Leu Val Ile Gln Gly Asp
   945                 950                 955                 960
   Glu Arg Met His Leu Pro Ser Pro Thr Asp Ser Asn Phe Tyr Arg Ala
                   965                 970                 975
   Leu Met Asp Glu Glu Asp Met Asp Asp Val Val Asp Ala Asp Glu Tyr
               980                 985                 990
   Leu Ile Pro Gln Gln Gly Phe Phe Ser Ser Pro Ser Thr Ser Arg Thr
           995                 1000                1005
   Pro Leu Leu Ser Ser Leu Ser Ala Thr Ser Asn Asn Ser Thr Val Ala
       1010                1015                1020
   Cys Ile Asp Arg Asn Gly Leu Gln Ser Cys Pro Ile Lys Glu Asp Ser
   1025                1030                1035                1040
   Phe Leu Gln Arg Tyr Ser Ser Asp Pro Thr Gly Ala Leu Thr Glu Asp
                   1045                1050                1055
   Ser Ile Asp Asp Thr Phe Leu Pro Val Pro Glu Tyr Ile Asn Gln Ser
               1060                1065                1070
   Val Pro Lys Arg Pro Ala Gly Ser Val Gln Asn Pro Val Tyr His Asn
           1075                1080                1085
   Gln Pro Leu Asn Pro Ala Pro Ser Arg Asp Pro His Tyr Gln Asp Pro
       1090                1095                1100
   His Ser Thr Ala Val Gly Asn Pro Glu Tyr Leu Asn Thr Val Gln Pro
   1105                1110                1115                1120
   Thr Cys Val Asn Ser Thr Phe Asp Ser Pro Ala His Trp Ala Gln Lys
                   1125                1130                1135
   Gly Ser His Gln Ile Ser Leu Asp Asn Pro Asp Tyr Gln Gln Asp Phe
               1140                1145                1150
   Phe Pro Lys Glu Ala Lys Pro Asn Gly Ile Phe Lys Gly Ser Thr Ala
           1155                1160                1165
   Glu Asn Ala Glu Tyr Leu Arg Val Ala Pro Gln Ser Ser Glu Phe Ile
       1170                1175                1180
   Gly Ala
   1185

## Description

### Field of the Invention

The invention relates to the treatment of proliferative disorders that are resistant to therapeutic agents that are ATP-competitive kinase inhibitors.

### Background of the Invention

### I. Resistance of Proliferative Disorders to ATP-competitive kinase inhibitors

Protein kinases have been shown to regulate cell proliferation. Inhibition of protein kinases has emerged as a research area that holds potential for development of new treatments for proliferative disorders, particularly cancer.

Inhibition of protein kinases has been accomplished therapeutically by administration of ATP-competitive small molecules. Such inhibitors block the enzymatic activity of kinases and thereby interfere with phosphorylation of cellular substrates. Examples of ATP-competitive small molecule inhibitors of kinases are shown in Table 1.

ATP-competitive kinase inhibitors have been shown to create selective pressures in target proliferating cells associated with the disorders for which the inhibitors are therapeutically administered. These selective pressures often result in the development of resistance in the target cells. Resistance may arise from mutation of the targeted kinase.

**Table 1: ATP-competitive small molecule inhibitors of kinases**

| Compound Name | Compound Structure |
|---|---|
| PD180970 | 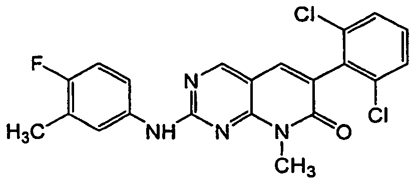 |
| BMS-354825 | 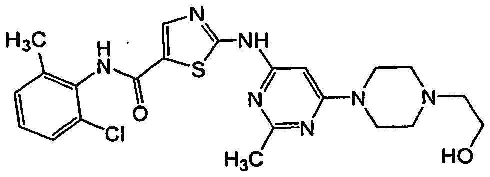 |
| Imatinib | 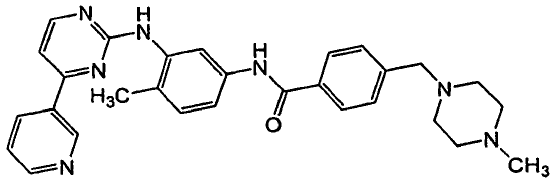 |
| SU5416 | 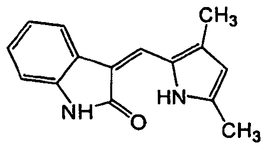 |
| SU6668 | 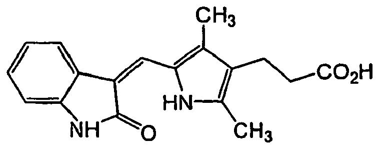 |
| SU11248 | 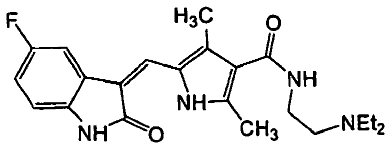 |
| AP23464 | 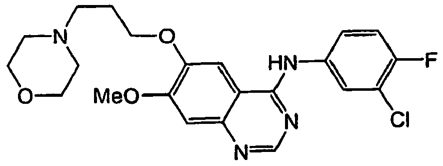 |
| Gefitinib | 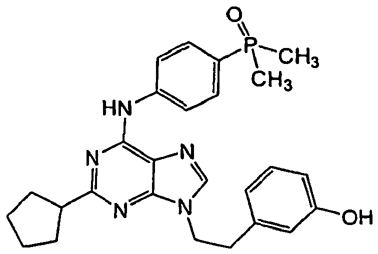 |
| Erlotinib | 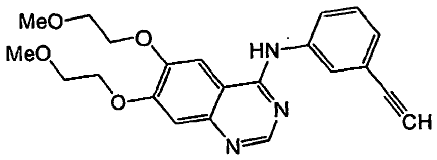 |
| PD153035 | 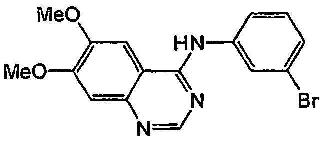 |
| SB 203580 | 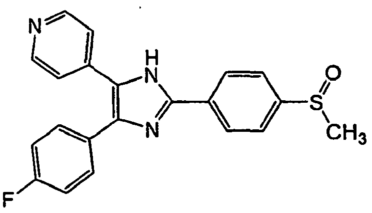 |

### A. BCR-ABL Associated Proliferative Disorders

### 1. Molecular Basis for the Disorder

Chronic myelogenous leukemia (CML), often referred to as chronic myeloid leukemia, was the first neoplastic disease to be associated with a specific chromosomal abnormality, namely the Philadelphia or Ph¹ chromosome. At the molecular level, the most notable feature is the translocation of the proto-oncogene *c*-*abl* from the long arm of chromosome 9 to the breakpoint cluster region (*bcr*) on chromosome 22, resulting in the formation of *bcr-abl* hybrid genes. The *c-abl* proto-oncogene normally encodes a protein (*ABL*) having tyrosine kinase activity. In cells carrying *bcr-abl* hybrid genes, the oncogene *BCR-ABL* is expressed. The tyrosine kinase activity of *BCR-ABL* is substantially augmented compared to the tyrosine kinase activity of *ABL.*

The BCR-ABL fusion protein commonly found in Philadelphia chromosome CML patients is a protein of approximately 210 kilodaltons (p210wt or p210BCR-ABL). The fusion transcript typically results from BCR exon 13 or 14 joined to ABL exon 2. (Chissoe et al (1995) Genomics 27:67-82). The ABL portion of the p210wt therefore consists of exons 2-11 of the c-ABL gene. The common convention in the literature to identify positions in the ABL tyrosine kinase portion in a particular BCR-ABL allele is to use the amino acid numbering for c-ABL which results from alternative splicing using exon 1a (protein ID AAB60394.1 and GenBank Accession number U07563). Therefore the mutations cited within this specification are likewise keyed to the amino acid numbering of this splice variant whose sequence is provided as SEQ ID NO: 1. The first 26 amino acids of SEQ ID NO: 1 are encoded by ABL exon 1a and are not found in the BCR-ABL fusion protein p210wt. The amino acid sequence encoded by exon 2 starts with E27 of proto-oncogene ABL which results from the transcript which results form the alternative splicing that uses exon 1a. The protein kinase domain is from approximately Ile 242 to amino acid 493. In this numbering system, the phosphate-binding loop (p-loop) is amino acids 249-256, the catalytic region is amino acids 361-367 and the activation loop is amino acids 380-402.

The ATP-competitive tyrosine kinase inhibitor imatinib is highly effective in inhibiting the tyrosine kinases *ABL, KIT* and platelet derived growth factor receptor (PDGFR). Therapeutic administration of imatinib demonstrates clinical efficacy in the treatment of CML and acute lymphoblastic lymphoma (ALL). *BCR-ABL* is also implicated in idiopathic pulmonary fibrosis, wherein inhibition of *ABL* by imatinib has been shown to prevent cell proliferation. Daniels et al., J. Clin. Invest., 114, 1308-16 (2004). However, a portion of imatinib-treated patients relapse when imatinib-resistant cells emerge.

### (2) Mutation of BCR-ABL Confers Imatinib Resistance

Imatinib resistance may result from kinase mutations at the site of imatinib binding. Mutations may occur in the kinase catalytic domain, which includes the so-called 'gatekeeper' residue, Thr315, and other residues that contact imatinib during binding (e.g., Phe317 and Phe 359). Other mutations occur in the ATP binding domain (the p-loop) and in the activation loop, an area of the kinase structure involved in a conformational change that occurs upon imatinib binding.

Imatinib binding to *BCR-ABL* involves formation of a hydrogen bond to the Thr315 hydroxyl group. The substitution of isoleucine for threonine at position 315 is one of the most frequent *BCR-ABL* mutations in imatinib-resistant CML. Alteration of Thr315 to the larger and non-hydrogen bonding isoleucine directly interferes with imatinib binding. Thr315, though necessary for binding of imatinib, is not required for ATP binding to *BCR-ABL.* Thus, the catalytic activity, and therefore the tumor-promoting function of the *BCR-ABL* oncoprotein, is preserved in the T315I mutant.

Imatinib binding is also affected by mutations in the kinase p-loop. *BCR-ABL* sequence analysis in relapsed CML and Ph+ ALL patients has detected p-loop mutations at Tyr253 and Glu255. Amino-acid substitutions at these positions may interfere with the distorted p-loop conformation required for imatinib binding. This is consistent with the observed *in vivo* resistance and the particularly poor prognosis of patients affected by *BCR-ABL* p-loop mutations such as Y253F and E255K (Branford et al., Blood, 102, 276-283 (2003).

Imatinib binding is associated with the inactive, unphosphorylated state of the *BCR-ABL* activation loop. Mutations in this region, such as H396R, destabilize a closed conformation of the activation loop and thereby counteract imatinib inhibition.

Another group of point mutations, remote from the imatinib binding site, lie in the carboxy-terminal lobe of the kinase domain. The most frequently detected *BCR-ABL* mutation falling into this group is M351T. The M351T mutation accounts for 15-20% of all cases of imatinib clinical resistance (Hochhaus et al., Leukemia, 18, 1321-31 (2004). M351T mutation appears to affect the precise positioning of residues in direct contact with imatinib (Cowan-Jacob et al., Mini Rev. Med. Chem., 4, 285-299 (2004), and Shah et al., Cancer Cell, 2, 117-125 (2002).

The above *BCR-ABL* mutations account for most of the *BCR-ABL* mutations which have been associated with imatinib resistance. However, a saturating mutational analysis of full-length *BCR-ABL* combined with a cellular screening procedure selecting for *BCR-ABL*-driven cell proliferation in the presence of imatinib, has revealed that mutations outside of the kinase domain can weaken kinase interaction with imatinib and thereby contribute to target resistance (Azam, et al., Cell, 112, 831-843 (2003).

*BCR-ABL* mutations clinically relevant to development of resistance to ATP-competitive kinase inhibitors such as imatinib include the following: G250E, F317L, Y253F, H396R, F311L, M351T, T315I, H396P, E255V, Y253H, Q252H, M244V, L387M, E355G, E255K and F359V. See, von Bubnoff et al., Leukemia 17, 829-838 (2003); Cowan-Jacob, et al., Mini Rev. Med. Chem. 4, 285-299 (2004); Hochhaus, et al., Leukemia 18, 1321-1331 (2004); Nardi, et al., Curr. Opin. Hematol. 11, 35-43 (2004); Ross, et al., Br. J. Cancer 90, 12-19; and Daub et al., Nature Reviews Drug Discovery, 3, 1001-10 (2004).

The therapeutic agents PD180970, BMS-354825 and AP23464 have demonstrated effectiveness against some imatinib-resistant proliferative disorders (Daub *et al., supra*). However, no agent has shown effectiveness against all *BCR-ABL* mutations. Furthermore, no agent is effective against resistance conferred by the T315I mutation.

### B. Platelet Derived Growth Factor (PDGF) Associated Proliferative Disorders

Platelet-derived growth factor receptor (PDGFR) is another tyrosine kinase. Molecular mechanisms, similar to the chromosomal translocation that generates the *BCR-ABL* oncoprotein, have been shown to create the imatinib-sensitive PDGFR fusion proteins PDGFRα (SEQ ID NO: 2) and PDGFRβ (SEQ ID NO: 3). PDGFRα and PDGFRβ are implicated in idiopathic hypereosinophilic syndrome and chronic myelomonocytic leukemia, respectively (Apperley et al., N. Engl. J. Med., 347, 481-487 (2002), and Cools et al., N. Engl. J. Med, 348, 1201-14 (2003). In hypereosinophilic syndrome, excessive proliferation of eosinophils often results from the constitutive tyrosine kinase activity of the chimeric Fip1-like (FIP1L1)-PDGFR protein, which arises from the fusion of the FIP1L1 and PDGFRα.

PDGFR and the proto-oncogene c-kit are implicated in malignant fibrous histocytoma (MFH). Imatinib has demonstrated inhibition of cell proliferation in three MFH cell lines (TNMY1, GBS-1 and Nara-F) that express one or both of the target kinases (Kawamoto et al., Anticancer Res., 24, 2675-9 (2004)).

PDGFRβ has also been implicated in prostate cancers, (Hofer et al., Neoplasia, 6, 503-12 (2004)), androgen dependent prostate cancers (Mathew et al., J. Clin. Oncol., 22, 3323-9 (2004)) and in dermatofibrosarcoma (Klener et al., Cas. Lek. Cesk., 143, 582-3 (2004)).

PDGFR and *BCR-ABL* are also implicated in endometrioid endometrial carcinoma (EEC) and uterine papillary serous carcinoma (UPSC) (Slomovitz et al., Gyneco. Oncol., 95,32-36 (2004)).

PDGFRβ has also been implicated in chordoma, and clinical administration of imatinib has been shown to have antitumor activity in a group of chordoma patients (Casali et al., Cancer, 1, 2086-97 (2004)).

PDGFRα and PDGFRβ are expressed in virtually all glioma cell lines and in fresh surgical isolates of human malignant astrocytoma. Imatinib has been shown to inhibit the growth of human glioblastoma cells (U343 and U87) *in vivo* in a nude mouse (Kilic et al., Cancer Res., 60, 5143-50 (2000)).

In one study, five out of five imatinib-treated patients with a detectable FIP1L1-PDGFR gene fusion experienced complete hematological remission (Cools *et al., Id*). One of the five patients eventually relapsed and was found to be resistant to imatinib. The imatinib resistance was attributed to a point mutation in the αPDGFR αATP binding site. The threonine-to-isoleucine substitution (T674I) occurs at a position equivalent to the T315I mutation of the gatekeeper residue of *BCR-ABL.*

The gatekeeper residue of *BCR-ABL* corresponds to gatekeeper residues on several kinases, including PDGFR, *KIT,* EGFR, *SRC* and P38. Despite clinical advances in the treatment of kinase-associated disorders, clinically relevant mutations have been identified in the kinase domains of PDGFR, *KIT,* and EGFR. Several of these mutations correspond to mutations that occur in *BCR-ABL,* and include gatekeeper residues. Mutation of the gatekeeper residue to a larger residue, such as isoleucine or methionine, has been shown to confer resistance in the kinases p38, *SRC* and EGFR to ATP-competitive inhibitors SB203580, PP1 and PD153035, respectively (Eyers et al., Chem. Biol., 5, 321-328 (1998); Liu et al., Chem. Biol., 8, 257-266 (1999); and Blencke et al., J. Biol. Chem., 278, 15435-40 (2003).

### C. KIT Associated Proliferative Disorders

Gastrointestinal stromal tumors (GIST), which are mesenchymal tumors of the stomach and small intestine, express the *KIT* tyrosine kinase receptor (SEQ ID NO: 4). GIST cells often contain an activating point mutation in the *KIT* kinase domain which leads to a constitutive kinase. Expression of activating *KIT* mutations in mice has been shown to cause tumors similar to human GISTs (Sommer et al., Proc. Natl. Acad. Sci., 100, 6706-11 (2003). C-*kit* expression has also been implicated in medulloblastoma, a highly invasive primitive neuroectodermal tumor of the cerebellum, which is the most common childhood malignant central nervous system tumor. In a study of ten medulloblastoma tumor samples, all expressed c-*kit* (Chilton-Macneill et al., Pediatr. Dev. Pathol., 7, 493-8 (2004)). C-*kit* has also been shown to be expressed in many uterine leiomyosarcomas (Raspollini et al., Clin. Cancer Res., 10, 3500-3 (2004)).

Secondary resistance formation to imatinib has been reported in at least one GIST patient. The resistance was caused by a T670I mutation in the KIT kinase domain; the mutation site corresponds to the T315I gatekeeper residue of *BCR-ABL* (Demitri et al., N Engl. J. Med, 347, 472-480 (2002). The mutation was confined to a metastatic lesion of the solid tumor, which progressed during continued imatinib therapy while other lesions were still responding to imatinib treatment (Tamborini et al., Gastroenterology, 127, 294-299, (2004).

Another secondary *KIT* mutation, Y823D, has been demonstrated to emerge during imatinib therapy and confer significant imatinib resistance. This point mutation is at a position that corresponds to the major phosphorylation site Tyr393 within the *ABL* activation loop (Wakai et al., Br. J. Cancer, 90, 2059-61 (2004). Phosphorylation of this site in wild-type *ABL* serves to destabilize the inactive conformation of the activation loop (Schindler et al., Science, 289, 1938-42 (2000). It has been suggested that the Y823D *KIT* mutation may mimic the phosphorylated Tyr393 of *ABL*, thereby conferring similar imatinib resistance (Daub et al., Nature Reviews Drug Discovery, 3, 1001-10 (2004)).

Another mutation has been characterized in the *KIT* activation loop. The kinase-activating D816V mutation in the KIT protein confers primary imatinib resistance and has been identified as a cause of disease in human mastocytosis (Ma et al., Blood, 99, 2059-61 (2002).

### D. EGFR Associated Proliferative Disorders

Epidermal growth factor receptor (EGFR, SEQ ID NO: 5) is another tyrosine kinase. Gefitinib, an EGFR inhibitor, is approved for the treatment of advanced non-small-cell lung cancers (NSCLCs) that do not respond to established chemotherapy regimens (Muhsin et al., Nature Rev. Drug Discov., 2, 515-516 (2003) and Cohen et al., Clin. Cancer Res., 10, 1212-18 (2004). The C-to-T single-nucleotide mutation that leads to the imatinib-refractory T315I *BCR-ABL* mutant also dramatically desensitizes EGFR to gefitinib by replacing the corresponding gatekeeper residue Thr766 with a methionine residue (T766M) (Blenke et al., Chem. Biol., 11, 691-791 (2004), and Blenke et al., J. Biol. Chem, 278, 15435-40 (2003).

### II. Prior Attempts To Overcome Imatinib Resistance

Pyrido[2,3-d]pyrimidines such as PD180970 (Table 1) have been shown to suppress the cancer-promoting activity of *BCR-ABL* activation loop mutants such as H396P (La Rosée et al., Cancer Res., 62, 7149-53 (2002), and von Bubnoff et al., Cancer Res., 9, 1267-73 (2003)). These compounds also inhibit *BCR-ABL* which contains the clinically common p-loop mutations associated with Tyr253 or Glu255. PD180970 has also been shown to inhibit *BCR-ABL* which contains the M351T mutant, which mutation accounts for about 15-20% of imatinib-resistant CML cases (Kantarjian et al., Blood, 101, 473-475 (2003)).

*SRC*, the first proto-oncogenic protein described, is a non-receptor tyrosine kinase. The *SRC* and *ABL* inhibitors BMS-354825 and AP23464 (Table 1) have shown activity similar to pyrido[2,3-d]pyrimidines in inhibiting *BCR-ABL* containing certain clinically common mutations associated with imatinib resistance (Shah et al., Science, 305, 399-401 (2004) and O'Hare et al., Blood, 104, 2532-39 (2004).

### III. α,β -Unsaturated Sulfones, Sulfoxides and Sulfonamides

Certain α,β-unsaturated sulfones, sulfoxides and sulfonamides, particularly styrylbenzyl sulfones, styrylbenzyl sulfoxides and styryl sulfonamides have been shown to possess antiproliferative, radioprotective and chemoprotective activity. *See:* US patents 6,599,932, 6,576,675, 6,548,553, 6,541,475, 6,486,210, 6,414,034, 6,359,013, 6,201,154, 6,656,973 and 6,762,207. However, none of these α,β-unsaturated sulfones, sulfoxides and sulfonamides have been reported to have activity in the treatment of kinase-dependent proliferative disorders that are resistant to treatment with ATP-competitive kinase inhibitors.

Some advances have been made in overcoming drug resistance caused by kinase mutations. However, new therapies are needed which are capable of treating proliferative disorders, particularly cancers, that are resistant to treatment by ATP-competitive kinase inhibitors and capable of preventing the development of such resistance. In addition, new therapies are needed which are capable of preventing or delaying the emergence of proliferative disorders, particularly cancers, that are resistant to treatment by ATP-competitive kinase inhibitors.

### Summary of the Invention

According to one embodiment of the invention, there is provided a use of a compound for the manufacture of a medicament for treating a kinase-dependent proliferative disorder in an individual, particularly a cancer, that is resistant to treatment with an ATP-competitive inhibitor of a kinase selected from the group consisting of BCR-ABL, KIT, epidermal growth factor receptor, PDGF-α, PDGF-β, SRC and P38, which resistance results from a mutation of said kinase, wherein the compound is according to Formula I:
wherein: 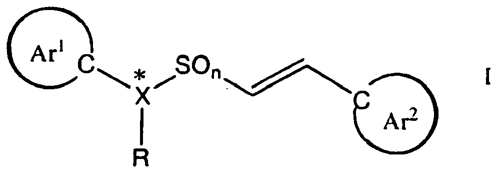 Ar¹ and Ar² are independently selected from substituted and unsubstituted aryl and substituted and unsubstituted heteroaryl;
X is N or CH;
n is 1 or 2;
R is -H or -(C₁-C₈)hydrocarbyl; and
* indicates that, when X is CH, and R is other than -H, the conformation of the substituents on the carbon atom of X is *R*-, *S*- or any mixture of *R*- and *S*-; or a pharmaceutically acceptable salt thereof; provided that when X is N, then n is 2.

According to another embodiment of the invention, use of a compound is provided for the manufacture of a medicament for preventing or delaying, in an individual suffering from a kinase-dependent proliferative disorder, the development of resistance to therapy which includes administration of an ATP-competitive inhibitor of a kinase selected from the group consisting of BCR-ABL, KIT, epidermal growth factor receptor, PDGF-α, PDGF-β, SCR and P38, which resistance results from a mutation of said kinase, wherein the compound is according to Formula I, as defined above. According to one embodiment, an effective amount of at least one ATP-competitive kinase inhibitor is administered.

The resistance of the kinase-dependent proliferative disorder to treatment with an ATP-competitive kinase inhibitor results from a mutation in the protein sequence of the kinase associated with the kinase-dependent proliferative disorder.

According to one embodiment of the invention, the kinase-dependent proliferative disorder that is treated is resistant to at least one ATP-competitive *BCR-ABL* inhibitor.

According to one embodiment of the invention, the resistance to an ATP-competitive inhibitor of *BCR-ABL* results from a mutation of one or more amino acid residues within a kinase domain of the *BCR-ABL* protein. According to some embodiments, the resistance arises from a mutation of at least one residue within the *BCR-ABL* p-loop. According to particular embodiments, the mutation comprises an alteration of *BCR-ABL* Tyr 253 or Glu255. According to other embodiments, the resistance arises from a mutation of at least one residue within the *BCR-ABL* activation loop. According to particular embodiments, the mutation is an alteration of His396.

According to a preferred embodiment, the ATP-competitive *BCR-ABL* inhibitor is imatinib.

According to other embodiments, the resistance to an ATP-competitive inhibitor of *BCR-ABL* results from a mutation within the *BCR-ABL* protein comprising at least one mutation selected from the group consisting of F317L, H396R, M35IT, H396P, Y253H, M244V, E355G, F359.V, G250E, Y253F, F311L, T315I, E255V, Q252H, L387M, E255K.

According to some embodiments of the invention, the kinase-dependent proliferative disorder that is resistant to treatment with an ATP-competitive kinase inhibitor is selected from the group consisting of chronic myelogenous leukemia, acute lymphoblastic lymphoma, idiopathic pulmonary fibrosis, idiopathic hypereosinophilic syndrome, chronic myelomonocytic leukemia, malignant fibrous histiocytoma, prostate cancers, androgen dependent prostate cancers, dermatofibrosarcoma, endometrioid endometrial carcinoma, uterine papillary serous carcinoma, chordoma, glioma, malignant astrocytoma, glioblastoma, gastrointestinal stromal tumors, medulloblastoma, uterine leiomyosarcomas, and non-small-cell lung cancers.

According to one embodiment of the invention, the kinase-dependent proliferative disorder is resistant to at least one ATP-competitive inhibitor of *KIT*, which resistance results from mutation of one or more amino acid residues within the *KIT* kinase domain.

According to certain embodiments, the mutation within the KIT protein comprises an alteration of at least one of Thr670, Tyr823 or Asp816. According to other embodiments, the mutation within the *KIT* protein comprises at least one mutation selected from the group consisting of T670I, Y823D and D816V.

According to one embodiment of the invention, the kinase-dependent proliferative disorder is resistant to at least one ATP-competitive inhibitor of EGFR, which resistance results from mutation of one or more amino acid residues within the EGFR kinase domain.

According to certain embodiments, the EGFR mutation comprises an alteration of Thr766. One such mutation is the mutation T766M.

According to another embodiment of the invention, the kinase-dependent proliferative disorder is resistant to at least one inhibitor of PDGFRα, which resistance results from mutation of one or more amino acid residues within the PDGFRα kinase domain.

According to certain embodiments, the PDGFRα mutation comprises an alteration of Thr674. One such mutation is the mutation is T674I.

According to another embodiment of the invention, the kinase-dependent proliferative disorder is resistant to at least one inhibitor of PDGFRβ, which resistance results from mutation of one or more amino acid residues within the PDGFRα kinase domain.

According to certain embodiments, the PDGFRβ mutation comprises an alteration of Thr681. One such mutation is the mutation is T681I.

According to an embodiment of the invention, substituents on substituted aryl or heteroaryl Ar¹ in Formula I may be independently selected from the group consisting of halogen, -(C₁-C₈)hydrocarbyl, -C(=O)R², NR²₂, NHC(=O)R³, NHSO₂R³, NH(C₂-C₆)alkylene-C(=O)R⁶, -NHCR²R⁴C(=O)R⁶, -C(=O)OR², -C(=O)NR²₂, -NO₂, -CN, -OR², -OC(=O)R³, -OSO₂R³, -O(C₂-C₆)alkylene-C(=O)R⁶, -OCR²R⁴C(=O)R⁶, -P(=O)(OR²)₂, -OP(=O)(OR²)₂, -O(C₂-C₆) alkylene)N(C₁-C₃)alkyl)₂, -NHC(=NH)NHR¹, -(C₁-C₆)haloalkyl, -O(C₁-C₆)haloalkyl; -SO₂NH₂; and -N=CH-R⁷; and substituents on substituted aryl or heteroaryl Ar² in Formula I may be independently selected from the group consisting of -(C₁-C₈)hydrocarbyl, -C(=O)R² halogen, -NO₂, -CN, -OR², -C(=O)OR², -NR²₂, -(C₁-C₆)haloalkyl; -SO₂NH₂; and -O(C₁-C₆)haloalkyl;
wherein:
each R² is independently selected from the group consisting of -H and -(C₁-C₈)hydrocarbyl;
each R³ is independently selected from the group consisting of -(C₁-C₈)hydrocarbyl, -O(C₁-C₈)hydrocarbyl, substituted and unsubstituted aryl, substituted and unsubstituted heterocyclyl(C₁-C₃)alkyl, substituted and unsubstituted heteroaryl(C₁-C₃)alkyl, -(C₂-C₁₀)heteroalkyl, -(C₁-C₆)haloalkyl, -CR²R⁴NHR⁵, -NR²₂, -(C₁-C₃)alkyleneNH₂, -(C₁-C₃)alkylene-N((C₁-C₃)alkyl)₂, -(C₁-C₃)perfluoroalkylene-N((C₁-C₃)alkyl)₂, -(C₁-C₃)alkylene-N⁺((C₁-C₃)alkyl)₃, -(C₁-C₃)alkylene-N⁺(CH₂CH₂OH)₃, -(C₁-C₃)alkylene-OR², -(C₁-C₄)alkylene-CO₂R², -(C₁-C₄)alkylene-C(=O)halogen, halo(C₁-C₃)alkyl-, -(C₁-C₃)alkylene-C(=O)(C₁-C₃)alkyl, and -(C₁-C₄)perfluoroalkylene-CO₂R²;
each R⁴ is independently selected from the group consisting of-H, -(C₁-C₆)alkyl, -(CH₂)₃-NH-C(NH₂)(=NH), -CH₂C(=O)NH₂, -CH₂CO₂R², -CH₂SH, -(CH₂)₂C(=O)-NH₂, -(CH₂)₂CO₂R², -CH₂-(2-imidazolyl), -(CH₂)₄-NH₂, -(CH₂)₂-S-CH₃, phenyl, -CH₂-phenyl, -CR₂-OH, -CH(OH)-CH₃, -CH₂-(3-indolyl), and -CH₂-(4-hydroxyphenyl);
each R⁵ is independently selected from the group consisting of -H, -C(=O)(C₁-C₇)hydrocarbyl and a carboxy terminally-linked peptidyl residue containing from 1 to 3 amino acids in which the terminal amino group of the peptidyl residue is present as a functional group selected from the group consisting of -NH₂, -NHC(=O)(C₁-C₆)alkyl, -NH(C₁-C₆)alkyl, -N(C₁-C₆ alkyl)₂ and -NHC(=O)O(C₁-C₇)hydrocarbyl;
each R⁶ is independently selected from the group consisting of -OR², -NR²₂, and an amino terminally-linked peptidyl residue containing from 1 to 3 amino acids in which the terminal carboxyl group of the peptidyl residue is present as a functional group selected from the group consisting of -CO₂R² and -C(=O)NR²₂; and
each R⁷ is independently selected from the group consisting of substituted and unsubstituted aryl and substituted and unsubstituted heteroaryl;
provided that the highest number of substituents on Ar¹ and Ar² is equal to the number of substitutable hydrogen atoms in the ring to which the substituents are attached.
Substituents on substituted aryl or heteroaryl groups that comprise R³ or R⁷ are preferably independently selected from the group consisting of -(C₁-C₆)alkyl, -(C₁-C₆)alkoxy, halogen, -C(=O)(C₁-C₆)alkyl, -NH₂, -NH(C₁-C₆)alkyl, -N(C₁-C₆)alkyl)₂, -NHC(=O)(C₁-C₆)alkyl, -NO₂, -CN, -(C₁-C₆)haloalkyl, -(C₁-C₆)alkylene-NH₂, -CO₂H, -CONH₂, -C(=N)NH₂, and heterocyclyl(C₁-C₆)alkyl; wherein heterocyclyl rings comprising heterocyclyl(C₁-C₆)alkyl are optionally substituted by -(C₁-C₆)alkyl or -C(=O)(C₁-C₆)alkyl.

Substituents on substituted heterocyclyl groups that comprise R³ are preferably independently selected from the group consisting of -(C₁-C₆)alkyl, -(C₁-C₆)alkoxy, halogen, -C(=O)(C₁-C₆)alkyl, -CO₂H, and -CONH₂.

According to some preferred embodiments of the invention, Ar¹ is phenyl. According to other preferred embodiments of the invention, Ar² is phenyl. According to still other preferred embodiments of the invention, both Ar¹ and Ar² are phenyl.

Preferably, when Ar¹ and Ar² are both phenyl, both Ar¹ and Ar² are at least monosubstituted.

According to some embodiments of the invention, the aryl and heteroaryl groups comprising Ar¹ and Ar² in Formula I compounds are mono-, di- or tri-substituted. According to other embodiments, the aryl and heteroaryl groups comprising Ar¹ and Ar² are substituted at all substitutable positions.

R is preferably -H or -(C₁-C₈)alkyl, most preferably -H or -(C₁-C₆)alkyl.

According to a preferred embodiment, substituents on substituted aryl or heteroaryl Ar¹ are independently selected from the group consisting of halogen, NR²₂, -NHCR²R⁴C(=O)R⁶, -OR², -OCR²R⁴C(-O)R⁶, and -OP(=O)(OR²)₂; and substituents on substituted aryl or heteroaryl Ar² are independently selected from the group consisting of -(C₁-C₈)hydrocarbyl, halogen, -OR², -C(=O)OR², and -NR²₂.

According to a more preferred embodiment, substituents on substituted aryl or heteroaryl Ar¹ are independently selected from the group consisting of halogen, NH₂, -NHCHR⁴C(=O)OR², -OH, -OCHR⁴C(=O) OR², and -OP(=O)(OR²)₂; and substituents on substituted aryl or heteroaryl Ar² are independently selected from the group consisting of -(C₁-C₆)alkyl, halogen, -OR², -C(=O)OR², and -NR²₂.

According to a most preferred embodiment, substituents on substituted aryl or heteroaryl Ar¹ are independently selected from the group consisting of halogen, -NH₂, -NHCH(CH₃)C(=O)OH, -NHCH₂C(=O)OH, -OH, -OCH(CH₃)C(=O)OH, and -OCH₂C(=O)OH; and substituents on substituted aryl or heteroaryl Ar² are independently selected from the group consisting of -(C₁-C₃)alkyl, halogen, -O(C₁-C₆)alkyl, -C(=O)OR², and -NR²₂.

Preferred compounds according to Formula I include, for example: (E)-2-(5-((2,4,6-trimethoxystyrylsulfonyl)methyl)-2-methoxyphenylamino)acetic acid; (racemic)-(*E*)-2-(5-((2,4,6-trimethoxystyrylsulfonyl)methyl)-2-methoxyphenylamino)propanoic acid; (*R*)-(*E*)-2-(5-((2,4,6-trimethoxystyrylsulfonyl)methyl)-2-methoxyphenylamino)propanoic acid; (*S*)-(*E*)-2-(5-((2,4,6-trimethoxystyrylsulfonyl)methyl)-2-methoxyphenylamino)propanoic acid; (*E*)-2-(5-((2,4,6-trimethoxystyrylsulfinyl)methyl)-2-methoxyphenylamino)acetic acid; (*E*)-2-(5-((2,4,6-trimethoxystyrylsulfinyl)methyl)-2-methoxyphenylamino)propanoic acid; (*E*)-2,4,6-trimethoxystyryl-*N*-(3-carboxymethylamino-4-methoxy-phenyl)sulfonamide; (*S*)-(*E*)-2-(5-((2,4,6-trimethoxystyrylsulfonyl)methyl)-2-methoxyphenylamino)-2-phenylacetic acid; (*R*)-(*E*)-2-(5-((2,4,6-trimethoxystyrylsulfonyl)methyl)-2-methoxyphenylamino)-2-phenylacetic acid; (racemic)-(*E*)-2-(5-((2,4,6-trimethoxystyrylsulfonyl)methyl)-2-methoxyphenylamino)-2-phenylacetic acid; (*E*)-2-(5-((2,4,6-trimethoxystyrylsulfonyl)methyl)-2-methoxyphenylamino)-2-methylpropanoic acid; (*E*)-1-(2-(4-bromobenzylsulfonyl)vinyl)-2,4-difluorobenzene; and pharmaceutically acceptable salts thereof.

According to certain embodiments of the invention, the compound according to Formula I comprises an isolated optical isomer, substantially free of the opposite enantiomer. According to one preferred sub-embodiment, the isolated optical isomer has the (*R*)- absolute configuration at the atom designated by *, and is substantially free of the (*S*)-enantiomer. According to another preferred sub-embodiment, the isolated optical isomer has the (*S*)- absolute configuration at the atom designated by *, and is substantially free of the (*R*)-enantiomer.

### Definitions

The term "individual" includes human beings and non-human animals.

The expression "effective amount" in connection with the treatment of a patient suffering from a proliferative disorder, particularly a cancer, refers to the amount of a compound of Formula I that inhibits the growth of cells that are proliferating at an abnormally high rate or alternatively induces apoptosis of such cells, preferably cancer cells, resulting in a therapeutically useful and selective cytotoxic effect on proliferative cells when administered to a patient suffering from a proliferative disorder, particularly a cancer. The term "effective amount" is inclusive of amounts of a compound of Formula I that may be metabolized to an active metabolite in an amount that inhibits the growth of abnormally proliferative cells or induces apoptosis of such cells.

The expression "proliferative disorder" means a disorder wherein cells are made by the body at an atypically accelerated rate. The expression "kinase-dependent proliferative disorder" refers to a proliferative disorder wherein the abnormally high cell proliferation is driven by the expression of a protein kinase.

The term "tumor" means an abnormal growth of tissue that results from abnormal cell proliferation, and which serves no physiological function. A tumor may be a benign tumor, *i.e*., a tumor which does not invade surrounding tissue and which does not otherwise endanger the life of the individual. A tumor may be cancerous. A cancerous tumor is malignant, *i.e.,* it tends to invade surrounding tissue and/or to metastasize, *i.e*., to spread to tissues in the body that are remote from the original tumor site.

The expression "kinase inhibitor" refers to an agent that acts to inhibit the kinase activity of a kinase.

The expression "ATP-competitive kinase inhibitor" means a kinase inhibitor that inhibits the kinase by competing with ATP for the ATP binding site on the kinase.

The term "alkyl", by itself or as part of another substituent means, unless otherwise stated, a straight, branched or cyclic chain saturated hydrocarbon radical, including di- and multi-radicals, having the number of carbon atoms designated in an expression such as (Cₓ-C_{y})alkyl. The expression (Cₓ-C_{y})alkyl wherein x < y, represents an alkyl chain containing a minimum of x carbon atoms and a maximum of y carbon atoms. Examples include: methyl, ethyl, propyl, isopropyl, butyl, isobutyl, tert-butyl, pentyl, neopentyl, hexyl, cyclohexyl and cyclopropylmethyl. Preferred is (C₁-C₃)alkyl, particularly ethyl, methyl and isopropyl.

The term "cycloalkyl" refers to alkyl groups that contain at least one cyclic structure. Examples include cyclohexyl, cyclopentyl, norbornyl, adamantyl and cyclopropylmethyl. Preferred is (C₃-C₁₂)cycloalkyl, particularly cyclopentyl, norbornyl, and adamantyl.

The term "alkylene" refers to a divalent alkyl radical having the number of carbon atoms designated (*i.e*. (C₁-C₆) means -CH₂-; -CH₂CH₂- -CH₂CH₂CH₂CH₂-; -CH₂CH₂CH₂CH₂CH₂-; and -CH₂CH₂CH₂-CH₂CH₂CH₂-, and also includes blanched divalent structures such as, for example, -CH₂CH₂CH₂-CH₂CH₂CH₂- and -CH(CH₃CH(CH₃)-, and divalent cyclic structures such as, for example 1,3-cyclopentyl.

The term "arylene", by itself or as part of another substituent means, unless otherwise stated, a divalent aryl medical. Preferred are divalent phenyl radicals, or "phenylene" groups, particularly 1,4-divalent phenyl radicals.

The term "heteroarylene", by itself or as part of another substituent means, unless otherwise stated, a divalent heteroaryl radical. Preferred are five- or six-membered monocyclic heteroarylene. More preferred are heteroarylene moieties comprising divalent heteroaryl rings selected from the group consisting of pyridine, piperazine, pyrimidine, pyrazine, furan, thiophene, pyrrole, thiazole, imidazole and oxazole, such as, for example 2,5-divalent pyrrole, thiophene, furan, thiazole, oxazole, and imidazole.

The term "alkoxy" employed alone or in combination with other terms means, unless otherwise stated, an alkyl group having the designated number of carbon atoms, as defined above, connected to the rest of the molecule via an oxygen atom, such as, for example, methoxy, ethoxy, 1-propoxy, 2-propoxy (isopropoxy) and the higher homologs and isomers. Preferred are (C₁-C₆)alkoxy, particularly ethoxy and methoxy.

The carbon chains in the alkyl and alkoxy groups which may occur in the compounds of the invention may be cyclic, straight or branched, with straight chain being preferred. The expression "(C₁-C₆)alkyl" thus extends to alkyl groups containing one, two, three, four, five or six carbons. The expression "(C₁-C₆)alkoxy" thus extends to alkoxy groups containing one, two, three, four, five or six carbons.

The term "hydrocarbyl" refers to any moiety comprising only hydrogen and carbon atoms. The term includes, for example, alkyl, alkenyl, alkynyl, aryl and benzyl groups. Preferred are (C₁-C₇)hydrocarbyl. More preferred are (C₁-C₆)alkyl and (C₃-C₁₂)cycloalkyl.

The term "heteroalkyl", by itself or in combination with another term means, unless otherwise stated, a stable straight or branched chain radical consisting of the stated number of carbon atoms and one or two heteroatoms selected from the group consisting of O, N, and S, and wherein the nitrogen and sulfur atoms may be optionally oxidized and the nitrogen heteroatom may be optionally quaternized. The heteroatom(s) may be placed at any position of the heteroalkyl group, including between the rest of the heteroalkyl group and the fragment to which it is attached, as well as attached to the most distal carbon atom in the heteroalkyl group. Examples include: -O-CH₂-CH₂-CH₃, -CH₂-CH₂-CH₂-OH, -CH₂-CH₂-NH-CH₃, -CH₂-S-CH₂-cH₂, and -CH₂CH₂-S(=O)-CH₃. Up to two heteroatoms may be consecutive, such as, for example, -CH₂-NH-OCH₃. or -CH₂-CH₂-S-S-CH₃.

The terms "halo" or "halogen" by themselves or as part of another substituent mean, unless otherwise stated, a fluorine, chlorine, bromine, or iodine atom.

The term "aromatic" refers to a carbocycle or heterocycle having one or more polyunsaturated rings having aromatic character ((4n + 2) delocalized π (pi) electrons). The term "aromatic" is intended to include not only ring systems containing only carbon ring atoms but also systems containing one or more non-carbon atoms as ring atoms. Systems containing one or more non-carbon atoms may be known as "heteroaryl" or "heteroaromatic" systems. The term "aromatic" thus is deemed to include "aryl" and "heteroaryl" ring systems.

The term "aryl" employed alone or in combination with other term, means, unless otherwise stated, a carbocyclic aromatic system containing one or more rings (typically one, two or three rings) wherein such rings may be attached together in a pendent manner, such as a biphenyl, or may be fused, such as naphthalene Examples include phenyl, anthracyl, and naphthyl, which may be substituted or unsubstituted. The aforementioned listing of aryl moieties is intended to be representative, not limiting.

The term "heterocycle" or "heterocyclyl" or "heterocyclic" by itself or as part of another substituent means, unless otherwise stated, an unsubstituted or substituted, stable, monocyclic or polycyclic heterocyclic ring system which consists of carbon atoms and at least one heteroatom selected from the group consisting of N, O, and S, and wherein the nitrogen and sulfur heteroatoms may be optionally oxidized, and the nitrogen atom may be optionally quaternized. The heterocyclic system may be attached, unless otherwise stated, at any heteroatom or carbon atom which affords a stable structure.

Heterocyclyl groups are inclusive of monocyclic and polycyclic heteroaryl groups and monocyclic and polycyclic groups that are not aromatic, such as saturated and partially saturated and monocyclic and polycyclic partially saturated monocyclic and polycyclic groups.

The term "heteroaryl" or 'heteroaromatic" refers to a heterocycle having aromatic character, and includes both monocyolic heteroaryl groups and polycyclic heteroaryl groups. A polycyclic heteroaryl group may include one or more rings which are partially saturated.

Examples of monocyclic heteroaryl groups include: Pyridyl; pyrazinyl; pyrimidinyl, particularly 2- and 5-pyrimidinyl pyridazinyl; thienyl; furyl; pyrrolyl, particularly 2-pyrrolyl and 1-alkyl-2-pyrrolyl; imidazolyl, particularly 2-imidazolyl; thiazolyl, particularly 2-thiazolyl; oxazolyl, particularly 2-oxazolyl; pyrazolyl, particularly 3- and 5-pyrazolyl, isothiazolyl, 1,2,3-triazolyl, 1,2,4-triazolyl, 1,3,4-triazolyl, tetrazolyl, 1,2,3-thiadiazolyl, 1,2,3-oxadiazolyl, 1,3,4-thiadiazolyl; and 1,3,4-oxadiazolyl.

Examples of monocyclic heterocycles that are not aromatic include saturated monocyclic groups such as: Aziridine, oxirane, thiirane, azetidine, oxetane, thietane, pyrrolidine, pyrroline, imidazoline, pyrazolidine, dioxolane, 1,4-dioxane, 1,3-dioxane, sulfolane, tetrahydrofuran, thiophane, piperazine, morpholine, thiomorpholine, tetrahydropyran, homopiperazine, homopiperidine, 1,3-dioxepane, hexamethyleneoxide and piperidine; and partially saturated monocyclic groups such as: 1,2,3,6-tetrahydropyridine, 1,4-dihydropyridine, 2,3-dihydrofuran, 2,5-dihydrofuran, 2,3-dihydropyran, 1,2-dihydrothiazole, 1,2-dihydrooxazole, 1,2-dihydroimidazole and 4,7-dihydro-1,3-dioxepin.

Examples of polycyclic heteroaryl groups include: indolyl, particularly 3-, 4-, 5-, 6- and 7-indolyl, quinolyl, isoquinolyl, particularly 1- and 5-isoquinolyl, cinnolinyl, quinoxalinyl, particularly 2- and 5-quinoxalinyl, quinazolinyl, phthalazinyl, 1,8-naphthyridinyl, 1,4-benzodioxanyl, coumarin benzofuryl, particularly 3-, 4-, 1,5-naphthyridinyl, 5-, 6- and 7-benzofuryl, 1,2-benzisoxazolyl, benzothienyl, particularly 3-, 4-, 5-, 6-, and 7-benzothienyl, benzoxazolyl, benzthiazolyl, particularly 2-benzothiazolyl and 5-benzothiazolyl, purinyl benzimidazolyl, particularly 2-benzimidazolyl, benztriazolyl, thioxanthinyl, carbazolyl, carbolinyl, acridinyl, pyrrolizidinyl, tetrahydroquinolyl; 1,2,3,4-tetrahydroisoquinolyl, dihydrocoumarinyl; 2,3-dihydrobenzofuryl; 2,3-dihydrobenzothienyl, N-methyl-2-indolinyl; and indolinyl.

Examples of non-aromatic polycyclic heterocycles include: pyrrolizidinyl and quinolizidinyl.

The aforementioned listing of non-aromatic heterocyclic moieties and heteroaryl moieties is intended to be representative, not limiting.

Preferred heteroaryl groups are 2-, 3- and 4-pyridyl; pyrazinyl; 2- and 5-pyrimidinyl; 3-pyridazinyl; 2- and 3-thienyl; 2- and 3-furyl; pyrrolyl; particularly N-methylpyrrol-2-yl; 2-imidazolyl; 2-thiazolyl; 2-oxazolyl; pyrazolyl; particularly 3- and 5-pyrazolyl; isothiazolyl; 1,2,3-triazolyl; 1,2,4-triazolyl; 1,3,4-triazolyl; tetrazolyl, 1,2,3-thiadiazolyl; 1,2,3-oxadiazolyl; 1,3,4-thiadiazolyl and 1,3,4-oxadiazolyl; indolyl, particularly 2-, 3-, 4-, 5-, 6- and 7-indolyl; cinnolinyl; quinoxalinyl, particularly 2- and 5-quinoxalinyl; quinazolinyl, particularly 2-, 5-, 6-, 7- and 8-quinazolinyl; phthalazinyl; 1,8-naphthyridinyl; 1,5-naphthyridinyl, particularly 1,5-naphthyridin-3-yl and 1,5-naphthyridin-4-yl; 1,4-benzodioxanyl; coumarinyl; benzofuryl, particularly 2-, 3- 5-, 6-and 7-benzofuryl; 1,2-benzisoxazolyl; benzothienyl, particularly 2-, 3-, 4-, 5-, 6-, and 7-benzothienyl; benzoxazolyl; benzthiazolyl, particularly 2-benzothiazolyl and 5-benzothiazolyl; purinyl; benzimidazolyl, particularly 2-benzimidazolyl; benztriazolyl; thioxanthinyl; carbazolyl; carbolinyl; and acridinyl, particularly 6-acridinyl.

More preferred heteroaryl groups are 2, 3- and 4-pyridyl; 2- and 3-thienyl; 2- and 3-furyl; 2-pyrrolyl; 2-imidazolyl; 2-thiazolyl; 2-oxazolyl; 2- and 3-indolyl; 2-, and 3-benzofuryl; 3-(1,2-benzisoxazolyl); 2-, and 3-benzothienyl; 2-benzoxazolyl; 1- and 2-benzimidazolyl, 2-, 3- and 4-quinolyl; and 2- and 5-benzthiazolyl. Most preferred heteroaryl groups are 2- and 3-indolyl; 2- and 3-pyrrolyl, 2-, and 3-benzofuryl; and 2-, and 3-benzothienyl.

The term "substituted" means that an atom or group of atoms has replaced hydrogen as the substituent attached to another group. For aryl and heteroaryl groups, the term "substituted" refers to any level of substitution, namely mono-, di-, tri-, tetra-, or penta-substitution, where such substitution is permitted. The substituents are independently selected, and substitution may be at any chemically accessible position.

The α,β-unsaturated (aryl or heteroaryl) sulfones, sulfoxides and sulfonamides are characterized by isomerism resulting from the presence of a double bond. This isomerism is commonly referred to as cis-trans isomerism, but the more comprehensive naming convention employs *E* and *Z* designations. The compounds are named according to the *Cahn-Ingold-Prelog* system, the IUPAC 1974 Recommendations, Section E: Stereochemistry, in Nomenclature of Organic Chemistry, John Wiley & Sons, Inc., New York, NY, 4th ed., 1992, p. 127-138. Using this system of nomenclature, the four groups about a double bond are prioritized according to a series of rules. Then, that isomer with the two higher ranking groups on the same side of the double bond is designated Z (for the German word "zusammen", meaning together). The other isomer, in which the two higher-ranking groups are on opposite sides of the double bond, is designated *E* (for the German word "entgegen", which means "opposite"). Thus if the four groups on a carbon-carbon double bond are ranked with A being the lowest rank and D being highest, A > B > C > D, the isomers would be named as in Scheme 1.

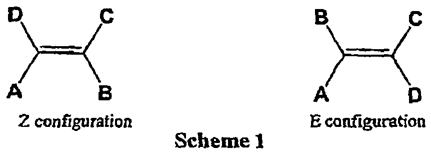

The present invention contemplates α,β-unsaturated (aryl or heteroaryl) sulfones, sulfoxides and sulfonamides in the E-configuration.

Some of the α,β-unsaturated (aryl or heteroaryl) sulfones, sulfoxides and sulfonamides according to Formula I may be characterized by isomerism resulting from the presence of a chiral center at X, when X is CH and R is other than -H. The isomers resulting from the presence of a chiral center comprise a pair of nonsuperimposable isomers that are called "enantiomers." Single enantiomers of a pure compound are optically active, *i.e*., they are capable of rotating the plane of plane polarized light. Single enantiomers are designated according to the Cahn-Ingold-Prelog system. March, Advanced Organic Chemistry, 4th Ed., (1992), p. 109. Once the priority ranking of the four groups is determined, the molecule is oriented so that the lowest ranking group is pointed away from the viewer. Then, if the descending rank order of the other groups proceeds clockwise, the molecule is designated (*R*) and if the descending rank of the other groups proceeds counterclockwise, the molecule is designated (*S*). In the example shown in Scheme 2 below, the *Cahn-Ingold-Prelog* ranking is A > B > C > D. The lowest ranking atom, D is oriented away from the viewer.

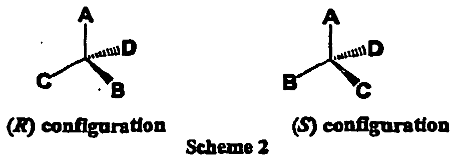

Unless otherwise indicated, both absolute configurations and mixtures thereof are included in the scope of α,β-unsaturated (aryl or heteroaryl) sulfones, sulfoxides and sulfonamides of Formula L.

The expression "substantially free" of the (*R*)- or (*S*)-enantiomer, when used to refer to an optically active compound according to Formula I, means the (*R*)- and (*S*)-enantiomers of the compound have been separated such that the composition is 80% or more by weight a single enantiomer. Preferably, the composition is 90% or more by weight a single enantiomer. More preferably, the composition is 95% or more by weight a single enantiomer. Most preferably, the composition is 99% or more by weight a single enantiomer.

Thus, by an "(*R*)-enantiomer of a compound according to Formula I substantially free of the (*S*)-enantiomer" is meant that the compound comprises 80% or more by weight of its (*R*)-enantiomer and likewise contains 20% or less of its (*S*)-enantiomer as a contaminant, by weight.

Isolated optical isomers may be purified from racemic mixtures by well-known chiral separation techniques. According to one such method, a racemic mixture of a compound having the structure of Formula I, or a chiral intermediate thereof, is separated into 99% wt% pure optical isomers by HPLC using a suitable chiral column, such as a member of the series of DAICEL CHIRALPAK family of columns (Daicel Chemical Industries, Ltd., Tokyo, Japan). The column is operated according to the manufacturer's instructions.

For compounds according to Formula I, more than one chiral center may be present in a molecule. Two pairs of enantiomers result from the presence of two chiral centers. Only the relationship between the mirror-image isomers is termed enantiomeric. The relationship between a single enantiomers and other isomers that exist as a result of additional chiral centers is termed "diastereomeric." Diastereomeric pairs may be resolved by known separation techniques including normal and reverse phase chromatography, and crystallization. Compounds of the present invention according to Formula I which contain two chiral centers, are understood to encompass isolated diastereomers, *e.g*., (*R,R*), (*R,S*), (*S,R*), and (*S,S*); isolated diastereomeric pairs, *e.g.,* (*R,R*) and (*R,S*), or (*S,R*), and (*S,S*); and all mixtures of diastereomers in any proportions.

Nomenclature employed herein for providing systematic names for compounds disclosed herein may be derived using the computer program package, Chemdraw^{®}, CambridgeSoft Corporation, Cambridge, MA 02140.

### Description of the Figures

Fig. 1 is a graph of the effect of Compound 1, imatinib or saline on *in vivo* growth of 32D/*BCR-ABL*^{T315I} cells in nude mice following intravenous injection of 1x10⁶ of the cells. Data is plotted as the average number of 32D/*BCR-ABL*^{T315I} cells per 10 fields +/-SEM (n=10).
Fig. 2 is a plot of the body weight of individual mice dosed with Compound 1 (100mg/kg, imatinib (100mg/kg or saline. The weight of each mouse in the three dose groups was determined daily. The average body weights were plotted as a percent of starting body weight
Fig. 3 is a graph of hematopoietic colony formation in CD-1 mice injected intravenously (tail vein injection) with saline or Compound 1 (100mg/kg) dissolved in saline.
Fig. 4(a) is a plot of the percent viable CML K562 cells remaining after a 72 hour incubation with varying concentrations of Compound 1 or imatinib.
Fig. 4(b) is a plot of the percent viable murine 32Dc13.BCR-*ABL* cells after a 72 hour incubation with varying concentrations of Compound 1 or imatinib.
Fig, 5(a) is a plot of the percent viable *BCR-ABL* T315L-expressing cells retaining after a 72 hour incubation with varying concentrations of Compound 1 or imatinib.
Fig. 5(b) is a plot of the percent viable *BCR-ABL* E255K-expressing cells remaining after a 72 hour incubation with varying concentrations of Compound I or imatinib.
Fig. 5(c) is a plot of the percent viable *BCR-ABL* Y253H-expressing cells after a 72 hour incubation with varying concentrations of Compound 1 or imatinib.
Fig. 5(d) is a plot of the percent viable *BCR-ABL* G250E-expressing cells after a 72 hour incubation with varying concentrations of Compound 1 or imatinib.
Fig. 6 is a graph of growth of 32Dc13 cells transfected to express wild-type or imatinib-resistant forms of *BCR-ABL* and treated with Compound 2.
Fig. 7 is a plot of the percent viable cells expressing wild type *BCR-ABL,* or the *BCR-ABL* mutations G250E, T3151 or M351T, after incubation with varying concentrations of Compound 4 for 72 hours.

### Detailed Description of the Invention

As described herein, certain α,β-unsaturated sulfones, sulfoxides and sulfonamides, or pharmaceutically acceptable salts thereof, are effective in inhibiting proliferation of target cells in kinase-dependent proliferative disorders that are resistant to treatment with ATP-competitive kinase inhibitors.

The compounds may be used in treating proliferative disorders in individuals whose disorder has become resistant to treatment with ATP-competitive kinase inhibitors due to mutations in the target kinase. For example, the compounds of the invention may be administered to treat imatinib-resistant CML and ALL, arising from point mutations in the target kinase, *BCR-ABL.* The compounds of the invention may be employed against other kinase-dependent proliferative disorders which have become refractory to therapy due to kinase mutations, such as proliferative disorders characterized by expression of *KIT*, PDGFRα, PDGFRβ, EGFR, *SRC* and P38. Mutations in these kinases have been shown to inhibit binding of imatinib and other ATP-competitive kinase inhibitors.

Proliferative disorders which may be driven by kinase expression include, for example, chronic myelogenous leukemia, acute lymphoblastic lymphoma, idiopathic pulmonary fibrosis, idiopathic hypereosinophilic syndrome, chronic myelomonocytic leukemia, malignant fibrous histiocytoma, prostate cancers, androgen dependent prostate cancers, dermatofibrosarcoma, endometrioid endometrial carcinoma, uterine papillary serous carcinoma, chordoma, glioma, malignant astrocytoma, glioblastoma, gastrointestinal stromal tumors, medulloblastoma, uterine leiomyosarcomas, and non-small-cell lung cancer.

### I. Identification of Proliferative Disorders Resistant to ATP-Competitive Kinase Inhibitors.

Mutations within the *BCR-ABL* kinase domain in imatinib-treated chronic myeloid leukemia (CML) are the main mechanism of acquired resistance to imatinib in CML. Likewise, mutations within the kinase domain of genes encoding for other kinases, *e.g.,* PDGFR, KIT, EGFR, SRC and P38, have been implicated as a primary mechanism for acquired resistance to protein kinase inhibitors which have antiproliferative activity versus the unmutated kinases.

Intervention in cases of drug resistance, by employing the method of the present invention, may be carried out following detection of kinase mutations in cells of the subject, particularly neoplastic cells of the subject. Mutations may be detected, for example, by sequencing the subject's relevant kinase genes in target cells, such as neoplastic cells, and comparing the sequence against a databank of resistance-conferring conferring mutations. In addition to direct DNA sequencing, the following methods known to those skilled in the art may also be used to detect kinase mutations: Single-strand Conformation Polymorphism (SSCP); Denaturing Gradient Gel Electrophoresis (DGGE); Denaturing High-Performance Liquid Chromatography (DHPLC); Chemical Mismatch Cleavage (CMC); Enzyme Mismatch Cleavage (EMC); Heteroduplex analysis; and the use of DNA microarrays.

According to one embodiment of the invention, quantitative polymerase chain reaction (RQ-PCR) of *BCR-ABL* mRNA in imatinib-treated subjects may be used to detect patients at risk of resistance. A significant portion of imatinib-treated subjects displaying a two-fold or more increase in *bcr-abl* expression have detectable *BCR-ABL* mutations, indicating that such a rise in *BCR-ABL* may serve as a primary indicator to test patients for imatinib-deactivating *BCR-ABL* kinase domain mutations (Branford et al., Blood, 104, 2926-32 (2004)). Elevated *BCR-ABL* expression in the cells of a subject undergoing ATP-competitive kinase inhibitor therapy would suggest the need for mutation analysis to identify possible resistance-conferring *BCR-ABL* mutations, particularly in the *BCR-ABL* kinase domain.

### II. Administration of Compounds

The compounds may be administered by any route, including oral and parenteral administration. Parenteral administration includes, for example, intravenous, intramuscular, intraarterial, intraperitoneal, intranasal, rectal, intravaginal, intravesical (*e.g*., to the bladder), intradermal, topical or subcutaneous administration. Also contemplated within the scope of the invention is the instillation of drug in the body of the patient in a controlled formulation, with systemic or local release of the drug to occur at a later time. For example, the drug may localized in a depot for controlled release to the circulation, or for release to a local site of tumor growth.

One or more compounds of Formula I may be administered simultaneously, by the same or different routes, or at different times during treatment.

Where both a Formula I compound and an ATP-competitive kinase inhibitor are both administered to the patient, the preferred schedule and dose will be dictated by the preferred mode for the ATP-competitive kinase inhibitor. The course of treatment differs from individual to individual, and those of ordinary skill in the art can readily determine the appropriate dose and schedule of administration of the ATP-competitive kinase inhibitor in a given clinical situation.

The specific dose of Formula I compound to obtain therapeutic benefit for treatment of a proliferative disorder will, of course, be determined by the particular circumstances of the individual patient including, the size, weight, age and sex of the patient, the nature and stage of the proliferative disorder, the aggressiveness of the proliferative disorder, and the route of administration of the compound.

For example, a daily dosage of from about 0.01 to about 50 mg/kg/day may be utilized, more preferably from about 0.05 to about 25 mg/kg/day. Particularly preferred are doses from about 0.5 to about 10.0 mg/kg/day, for example, a dose of about 5.0 mg/kg/day. The dose may be given over multiple administrations, for example, two administrations of 2.5 mg/kg. Higher or lower doses are also contemplated.

When both a Formula I compound and an ATP-competitive kinase inhibitor are both administered to the patient, the routes of administration may be the same or different for the two drugs. The drugs may be administered on the same or different days. According to one embodiment, the drugs are administered on succeeding days. Administering the drugs on the same day encompasses simultaneous or virtually simultaneous administration, up to administration within an about 24 hour timeframe of each other.

Dosing regimens for leukemic proliferative diseases, most particularly for chronic myelogenous leukemia, may include the following.

For a CML patient in chronic phase who is imatinib-resistant due to a mutation in the kinase domain of the *bcr-abl* gene, treatment comprises a daily oral dose of a Formula I compound ranging from about 1.5 mg/kg/day to about 4 mg/kg/day. If the desired therapeutic result is not achieved after 3 months of treatment, the dose is increased in about 0.5 to 1.5 mg/kg/day increments with ongoing monitoring occurring every 3 months. Upon appearance of disease progression, the dose is increased in about 1.0 to 3.0 mg/kg/day increments until the desired therapeutic result is achieved.

For a CML patient in either accelerated phase or blast phase, treatment comprises a daily oral dose of a Formula I compound ranging from about 2.5 mg/kg/day to about 4.5 mg/kg/day. If the desired therapeutic result is not achieved after 3 months of treatment, the dose is increased in about 0.5 to 1.5 mg/kg/day increments with ongoing monitoring occurring every 3 months. Upon appearance of disease progression, the dose is increased in about 1.0 to 3.0 mg/kg/day increments until the desired therapeutic result is achieved.

For treatment of a CML patient who becomes imatinib-resistant during a course of treatment with imatinib at 400 mg daily, the imatinib dose is increased to 600 or 800 mg daily and a daily oral dose of a Formula I compound about 2.0 to 5.0 mg/kg/day is added. If the desired therapeutic result is not achieved after 3 months of treatment, the dose of the Formula I compound is increased in about 0.5 to 1.5 mg/kg/day increments with ongoing monitoring occurring every 3 months. Upon appearance of disease progression, the dose of the Formula I compound is increased in about 1.0 to 3.0 mg/kg/day increments until the desired therapeutic result is achieved.

As a first line therapy for CML patients, treatment comprises a combination of imatinib and a Formula I compound. A daily oral dose of 400 milligrams of imatinib and about 1.0 to 4.0 mg/kg/day of a Formula I compound is administered. If the desired therapeutic result is not achieved after 3 months of treatment, the dose of the Formula I compound is increased in about 0.5 to 1.5 mg/kg/day increments with ongoing monitoring occurring every 3 months. Upon disease progression, the imatinib dose is increased to 600 or 800 milligrams and the dose of the Formula I compound is increased in about 1.0 to 3.0 mg/kg/day increments until the desired therapeutic result is achieved.

In each of the regimens above, treatment is ongoing, assuming acceptable toxicity, until the patient expires or other factors, such as terminal stage disease, dictate cessation of the treatment. Monitoring frequency of disease status can be adjusted as necessary by one skilled in the art.

Monitoring of disease status is well known in the art. For monitoring of leukemic proliferative diseases, most particularly chronic myelogenous leukemia, disease markers include but are not limited to: blood counts, cytogenetic monitoring of bone marrow cells (e.g. percent of Philadelphia-positive metaphases) and molecular markers (e.g. amount of bcr-abl mRNA transcript measured for instance by RQ-PCR or molecular rearrangements by fluorescence in situ hybridization (FISH)).

### III. Pharmaceutical Compositions

The Formula I compounds may be administered in the form of a pharmaceutical composition, in combination with a pharmaceutically acceptable carrier. The active ingredient in such formulations may comprise from 0.1 to 99.99 weight percent. By "pharmaceutically acceptable carrier" is meant any carrier, diluent or excipient which is compatible with the other ingredients of the formulation and to deleterious to the recipient.

The active agent is preferably administered with a pharmaceutically acceptable carrier selected on the basis of the selected route of administration and standard pharmaceutical practice. The active agent may be formulated into dosage forms according to standard practices in the field of pharmaceutical preparations. Alphonso Gennaro, ed., Remington's Pharmaceutical Sciences, 18th Ed., (1990) Mack Publishing Co., Easton, PA. Suitable dosage forms may comprise, for example, tablets, capsules, solutions, parenteral solutions, troches, suppositories, or suspensions.

For parenteral administration, the active agent may be mixed with a suitable carrier or diluent such as water, an oil (particularly a vegetable oil), ethanol, saline solution, aqueous dextrose (glucose) and related sugar solutions, glycerol, or a glycol such as propylene glycol or polyethylene glycol. Solutions for parenteral administration preferably contain a water soluble salt of the active agent. Stabilizing agents, antioxidant agents and preservatives may also be added. Suitable antioxidant agents include sulfite, ascorbic acid, citric acid and its salts, and sodium EDTA. Suitable preservatives include benzalkonium chloride, methyl- or propyl-paraben, and chlorbutanol. The composition for parenteral administration may take the form of an aqueous or nonaqueous solution, dispersion, suspension or emulsion.

For oral administration, the active agent may be combined with one or more solid inactive ingredients for the preparation of tablets, capsules, pills, powders, granules or other suitable oral dosage forms. For example, the active agent may be combined with at least one excipient such as fillers, binders, humectants, disintegrating agents, solution retarders, absorption accelerators, wetting agents absorbents or lubricating agents. According to one tablet embodiment, the active agent may be combined with carboxymethylcellulose calcium, magnesium stearate, mannitol and starch, and then formed into tablets by conventional tableting methods.

### IV. Salts of Compounds According to Formula I

The Formula I compounds may take the form of salts. The term "salts", embraces addition salts of free acids or free bases which are compounds of the invention. The term "pharmaceutically acceptable salt" refers to salts which possess toxicity profiles within a range that affords utility in pharmaceutical applications.

Suitable pharmaceutically-acceptable acid addition salts may be prepared from an inorganic acid or from an organic acid. Examples of inorganic acids include hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, examples of which include formic, acetic, propionic, succinic, glycolic, gluconic, lactic, malic, tartaric, citric, ascorbic, glucuronic, maleic, fumaric, pyruvic, aspartic, glutamic, benzoic, anthranilic, salicylic, 4-hydroxybenzoic, phenylacetic, mandelic, pamoic, methanesulfonic, ethanesulfonic, benzenesulfonic, pantothenic, trifluoromethanesulfonic, 2-hydroxyethanesulfonic, toluenesulfonic, sulfanilic, cyclohexylaminosulfonic, stearic, alginic, γ-hydroxybutyric, galactaric and galacturonic acid.

Suitable pharmaceutically-acceptable base addition salts of Formula I compounds include for example, metallic salts including alkali metal, alkaline earth metal and transition metal salts such as, for example, calcium, magnesium, potassium, sodium and zinc salts. Pharmaceutically-acceptable base addition salts also include organic salts made from basic amines such as, for example, *N,N*'-dibenzylethylenediamine, chloroprocaine, choline, diethanolamine, ethylenediamine, meglumine (*N*-methylglucamine) and procaine.

All of these salts may be prepared by conventional means from the corresponding compound according to Formula I by reacting, for example, the appropriate acid or base with a the compound according to Formula L

### V. Preparation of α,β-Uunsaturated Sulfones, Sulfoxides and Sulfonamides Useful in the Practice of the Invention

α,β-Unsaturated sulfones **D1** (wherein Ar¹, Ar², R and * are as defined for Formula I compounds), may be prepared by Knoevenagel condensation of aromatic aldehydes with benzylsulfonyl acetic acids **C1**. The procedure is described by Reddy et al., Acta. Chim. Hung. 115:269-71 (1984); Reddy et al., Sulfur Letters 13:83-90 (1991); Reddy et al., Synthesis No. 4, 322-323 (1984); and Reddy et al., Sulfur Letters 7:43-48 (1987), the entire disclosures of which are incorporated herein by reference. The general synthesis according to a Knoevenagel condensation is depicted in Scheme 3 below.

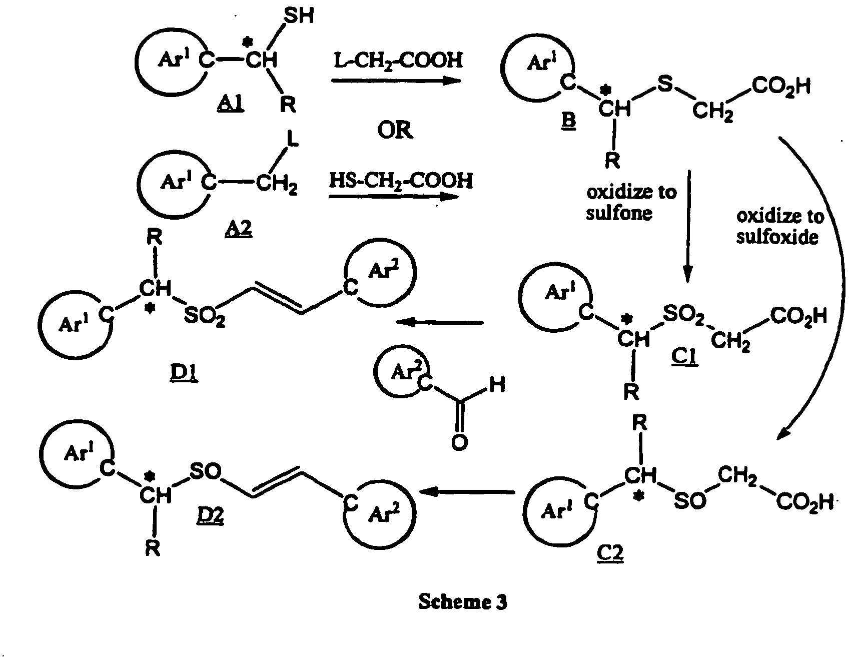

The starting benzylsulfonyl acetic acids **C1**, employed in Scheme 3 may be prepared by oxidation of the corresponding benzylmercaptoacetic acids **B**. The oxidation may be accomplished using any oxidation conditions suitable to oxidize a sulfide to a sulfone. Suitable reagents include peroxides such as hydrogen peroxide, peracids such as meta-chloroperoxybenzoic acid (MCPBA) and persulfates such as OXONE® (potassium peroxymonosulfate). The oxidation reaction is preferably carried out in the presence of a suitable solvent. Suitable solvents include, for example, water, acetic acid or non-polar solvents such as dichloromethane (DCM).

The benzylmercaptoacetic acids. **B** may be prepared by reacting mercaptoacetic acid with a benzyl compound **A2**, or by reacting a haloacetic acid with a benzyl mercaptan **A1**.

As depicted in Scheme 3, α,β-unsaturated sulfoxides may be prepared by the Knoevenagel condensation of aromatic aldehydes with benzylsulfinyl acetic acids, **C2.**

The starting benzylsulfinyl acetic acids **C2,** employed in Scheme 3 in the preparation of sulfoxides **D2,** may be prepared by oxidation of the corresponding benzylmercaptoacetic acids **B.** The oxidation may be accomplished using any oxidation conditions suitable to oxidize a sulfide to a sulfoxide. Suitable reagents include peroxides such as hydrogen peroxide, peracids such as meta-chloroperoxybenzoic acid (MCPBA) and persulfates such as OXONE® (potassium peroxymonosulfate). The reaction is preferably carried out in the presence of a suitable solvent. Suitable solvents include, for example, water, acetic acid or non-polar solvents such as dichloromethane (DCM). The oxidation to a sulfoxide is generally performed at reduced temperature, *e.g*., from about - 10° to about 25°C, and the oxidation reaction is monitored so as to terminate the reaction prior to over-oxidation to the sulfone.

α,β-Unsaturated sulfonamides **G** (wherein Ar¹, Ar², and R are as defined for Formula I), may be prepared by Knoevenagel condensation of aromatic aldehydes with arylaminosulfonylacetic acids **F,** as shown in Scheme 4.

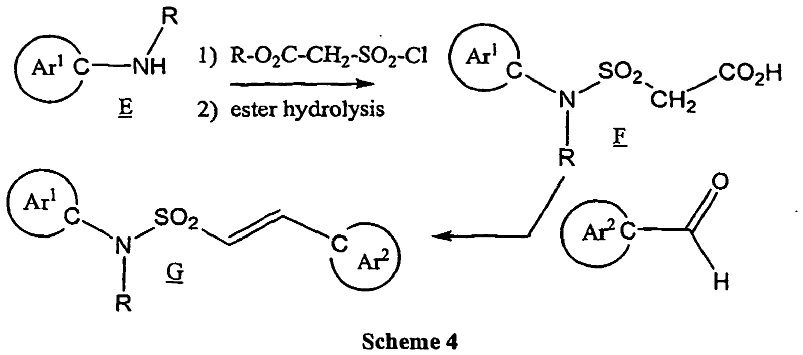

α,β-Unsaturated sulfonamides **G**, may alternately be prepared by reacting the aromatic amine **E**, with a aromatic sulfonylhalide **H** as depicted in Scheme 5. The aromatic sulfonylhalide **H** is preferably prepared by reacting an aromatic olefin, **J** with sulfuryl chloride.

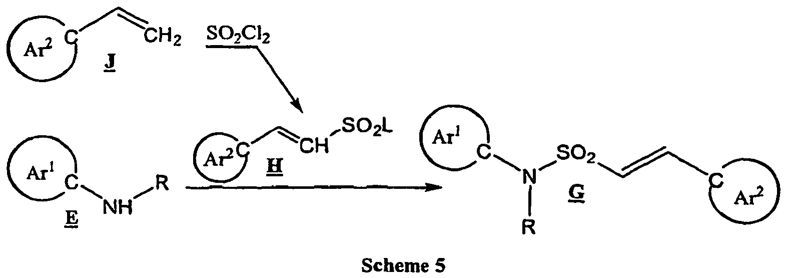

The preparation of compounds of Formula I is also described in one or more of the following US patents, the entire disclosures of which are incorporated herein by reference: 6,599,932, 6,576,675, 6,548,553, 6,541,475, 6,486,210, 6,414,034, 6,359,013, 6,201,154, 6,656,973 and 6,762,207.

The practice of the invention is illustrated by the following non-limiting examples. In the examples:
"Compound 1" is (*E*)-2-(5-((2,4,6-trimethoxystyrylsulfonyl)methyl)-2-methoxyphenylamino) propanoic acid: 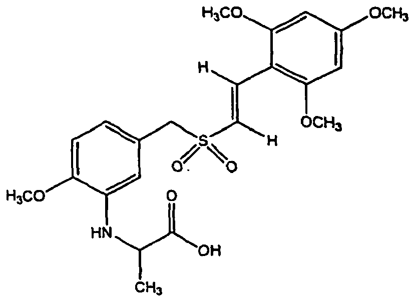
"Compound 2" is (*E*)-2-(5-((2,4,6-trimethoxystyrylsulfonyl)methyl)-2-methoxyphenylamino) acetic acid: 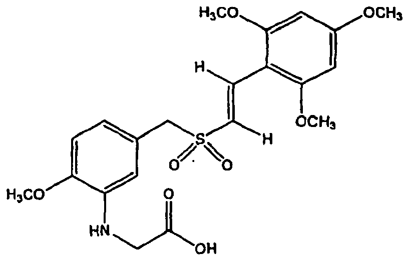
"Compound 3" is the sodium salt of (*E*)-2-(5-((2,4,6-trimethoxystyrylsulfonyl)methyl)-2-methoxyphenylamino)-2-methylpropanoic acid: 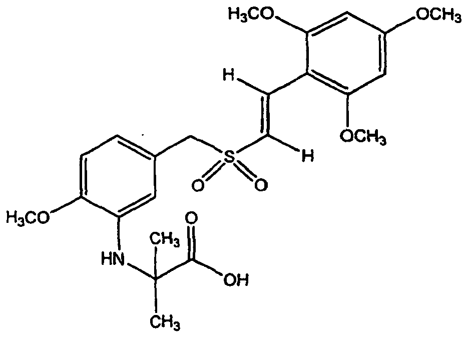
"Compound 4" is (*E*)-1-(2-(4-bromobenzylsulfonyl) vinyl)-2,4-difluorobenzene:

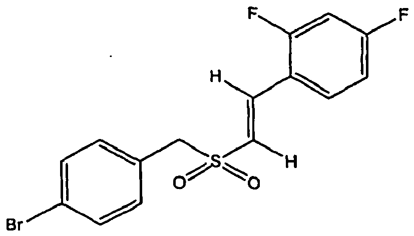

### Examples

### Example 1: Effect of (E)-2-(5-((2,4,6-trimethoxystyrylsulfonyl)methyl)-2-methoxy-phenylamino)propanoic acid (Compound 1) on the In Vivo Growth of Cells Expressing the Imatinib Resistant BCR-ABL^{T315I}.

### A. 32Dc13 Cell line

32Dc13 Cells (Rovera et al., Oncogene, 1, 29-35 (1987)) were maintained in Iscove's Modified Dulbecco's Medium supplemented with 10% FBS, 1U/mL penicillin-streptomycin and 10% WEHI-3B conditioned medium as a source of IL-3 (Ymer et al., Nature, 317, 255-258 (1985)).

### B. Generation of wild-type and imatinib-resistant mutants of BCR-ABL.

Oligonucleotides corresponding to published *bcr-abl* mutations (Shah et al., Oncogene 22, 7389-85 (2003)) associated with imatinib resistance were used to introduce these mutations into the full-length *bcr-abl* cDNA using PCR-based site-directed mutagenesis (Myers et al., PCR Technology, eds. Erlich, H.A., Stockton Press, London (1989)). All constructs were verified by sequence analysis. pCDNA3-based expression plasmids encoding wild-type and imatinib resistant forms of *BCR-ABL* were introduced into actively proliferating 32Dc13 cells by electroporation as previously described (Kumar et al., Mol. Cell. Biol., 23, 6631-45 (2003)) and cells selected in the absence of IL-3. The expression of the *BCR-ABL* proteins was determined, as described below, by Western blot and kinase assays.

### C. Analysis of in vivo growth of T315I cells

Female athymic nude mice (ncr/ncr) were injected intravenously with 1x10⁶ 32Dc13 cells expressing the mutant *BCR-ABL*^{T315I} via the tail vein. Treatments (3 groups with 10 mice per dosage group) were started 24 hours after cell injections by daily intraperitoneal injections of saline (vehicle), Compound 1 (100mg/kg) or imatinib (100 mg/kg). Imatinib injections were terminated after 10 days due to toxicity.

### (i) Toxicity of Imatinib and Compound 1

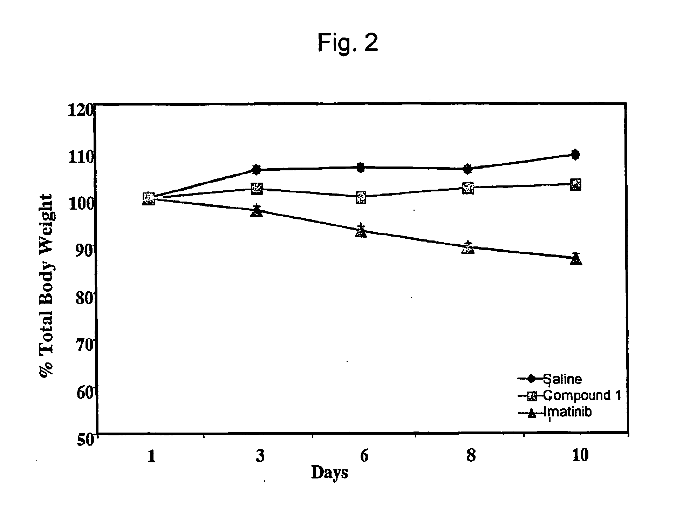

Body weights of the test animals were taken every two days. The body weight data is shown in Figure 2. The Compound 1 treated mice showed no signs of toxicity, such as body weight loss, ruffled coats, lethargy or abnormal feces. In contrast, administration of imatinib for 10 days produced severe toxicity as judged by greater than a 20% loss of bodyweight, which resulted in the termination of drug administration.

### (ii) Analysis of In vivo Growth of T315I cells

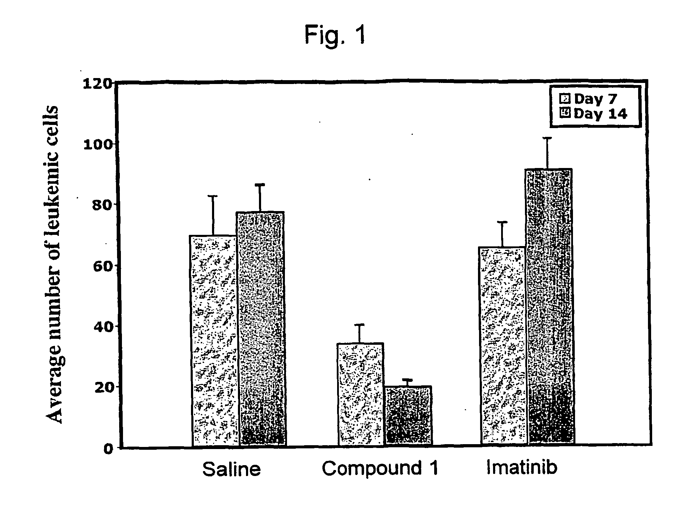

Blood smears on the treated mice were performed 7 and 14 days after the start of treatments. One drop of blood from each mouse was smeared onto glass slides, air dried and stained with modified Wright stain (Sigma). The T315I cells in the blood were easily visible due to their blue staining and size difference. The number of T315I cells per 10 fields was determined by counting 10 fields of view containing an equal density of red blood cells using a 40x objective on an upright Olympus microscope. The *in vivo* growth of T315I cells in the study are shown in Figure 1. The number of T315I cells in the blood of mice treated with Compound 1 was significantly reduced on days 7 and 14 as compared to the number of cells found in the vehicle and imatinib treated groups.

### Example 2: Hematopoietic Colony Formation Assay

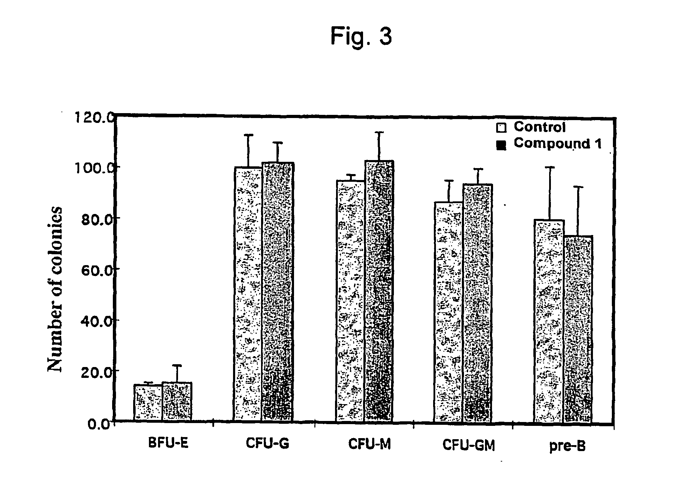

To examine the effects of Compound 1 on normal hematopoietic cell population *in vivo,* a dose of 100mg/kg of this compound in normal saline (or saline alone as a control) was injected intravenously (tail vein injection) into CD-1 mice (10 animals per group). The effect on the *in vitro* hematopoietic colony formation of normal bone marrow cells derived from these mice was determined 24 hrs following administration. Bone marrow cells were extracted from the mice after 24 hrs and 2x10⁵ cells were plated on methycellulose containing appropriate cytokines for lineage specific colony formation. Colonies were counted after 5 to 14 days of incubation. Data for the bone marrow colony formation assay is shown in Figure 3. No reduction in colony formation of the erythroid, myeloid or lymphoid lineages with administration of Compound 1.

### Example 3: Activity of Compound 1 against BCR-ABL expressing cell lines.

### A. Cell lines

K562 cells were maintained in RPMI (Roswell Park Memorial Institute) medium supplemented with 10% FBS and 1U/ml penicillin-streptomycin.

32Dc13 Cells (Rovera et al., Oncogene, 1, 29-35 (1987)) were maintained in Iscove's Modified Dulbecco's Medium supplemented with 10% FBS, 1U/mL penicillin-streptomycin and 10% WEHI-3B conditioned medium as a source of IL-3 (Ymer et al, Nature, 317, 255-258 (1985)).

### B. Cell Viability on Treatment with Imatinib or Compound 1

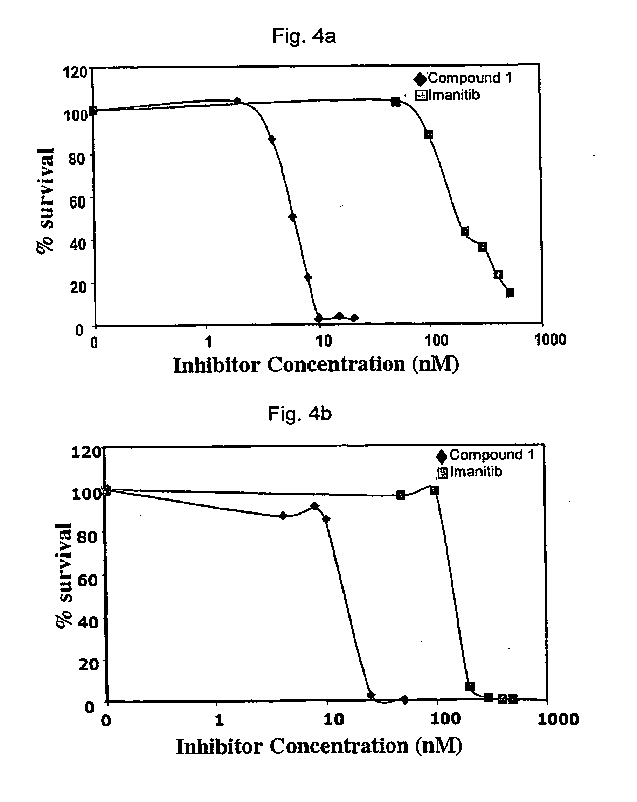

K562 cells, and murine 32Dc13.*BCR-ABL* cells that ectopically expressed the wild-type p210*^{BCR-ABL}* oncoprotein, were incubated with varying concentrations of Compound 1 for a period of 72 hours. Cell viability was then determined by trypan blue exclusion. The percent viable cells compared to vehicle-treated controls was determined. In both cell lines, incubation with Compound 1 resulted in a rapid loss of viability with an LD₅₀ of 10-15nM as shown in Figures 4a and 4b. The cell viability assay was also performed using imatinib. The IC₅₀ of imatinib was found to be greater than 100nM (Figure 4a and 4b). The data for imatinib agrees with published data (O'Dwyer, *Id*.).

Both Compound 1 and imatinib inhibit proliferation of *BCR-ABL*⁺ cells. Compound 1 inhibited proliferation of *BCR-ABL*⁺ cell lines at a concentration 10-fold less than that shown for imatinib.

### Example 4: Activity of Compound 1 Against Imatinib-resistant Tumor Cell Lines.

Mutations corresponding to those published in the literature (Shah. et al., Oncogene, 22, 7389-7395 (2003)) were introduced into the *bcr-abl* cDNA, which was then introduced into actively proliferating 32Dc13 cells (Rovera et al., Oncogene 1, 29-35 (1987)) according to the following procedure.

Oligonucleotides corresponding to published *ber-abl* mutations (Shah et al., Oncogene 22, 7389-85 (2003)) associated with imatinib resistance were used to introduce these mutations into the full-length *bcr-abl* cDNA using PCR-based site-directed mutagenesis (Myers et al., PCR Technology, eds. Erlich, H.A., Stockton Press, London (1989)). All constructs were verified by sequence analysis. pCDNA3-based expression plasmids encoding wild-type and imatinib resistant forms of *BCR-ABL* were introduced into actively proliferating 32Dc13 cells by electroporation as previously described (Kumar et al., Mol. Cell. Biol., 23, 6631-45 (2003)).

Transfectants were selected in the absence of IL-3. The expression and activity of each mutant *BCR-ABL* protein was confirmed by Western blot analysis and kinase assays (data not shown). All mutants conferred imatinib resistance at levels comparable to previously published studies when compared to wild-type p210*^{BCR-ABL}* expressing cells (data not shown) (Azam et al., Cell, 112, 831-843 (2003)).

The transfected cells were then cultured in the presence of various concentrations of Compound 1 or imatinib. The total number of viable cells was determined by trypan blue exclusion at 72 hours post-treatment The percent viable cells compared to vehicle-treated controls was determined. All of the transfected cell lines, including cells expressing the T315I mutant, were found to be extremely sensitive to the growth inhibitory activity of Compound 1. The sensitivity of the cell lines, expressed in terms of GI₅₀ (the concentration which achieved 50% growth inhibition), is listed in Table 2 for 16 clinically relevant *BCR-ABL* mutations.

**Table 2: Compound 1-induced Growth inhibition of Cells**

| Mutation | GI₅₀ (nM) | Mutation | LD₅₀ (nM) |
|---|---|---|---|
| None (wt) | 10 | G250E | 5.9 |
| F317L | 7.8 | Y253F | 6.8 |
| H396R | 6.8 | F311L | 6.8 |
| M351T | 7.8 | T315I | 7.5 |
| H396P | 7.9 | E255V | 7.8 |
| Y253H | 7.8 | Q252H | 7.0 |
| M244V | 7.8 | L387M | 7.1 |
| B355G | 7.8 | E255K | 8.4 |
| F359V | 7.1 | | |

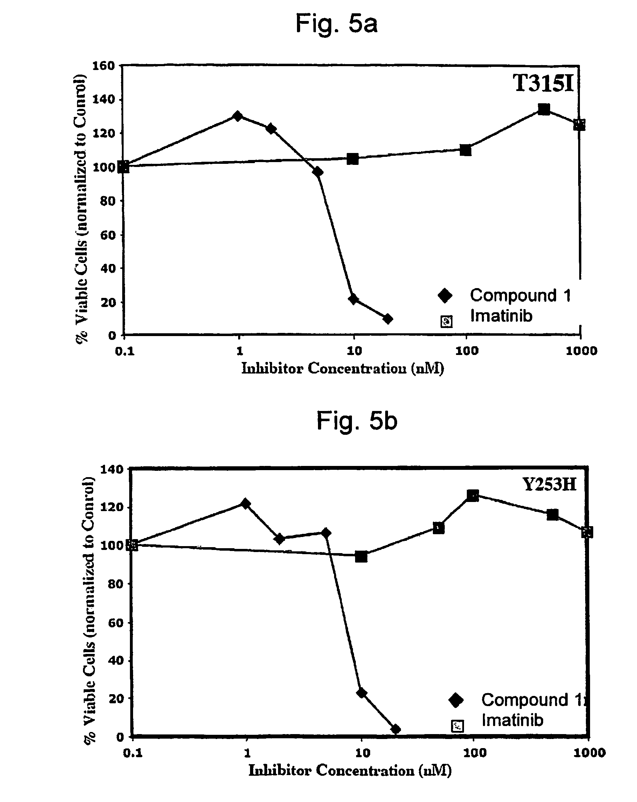

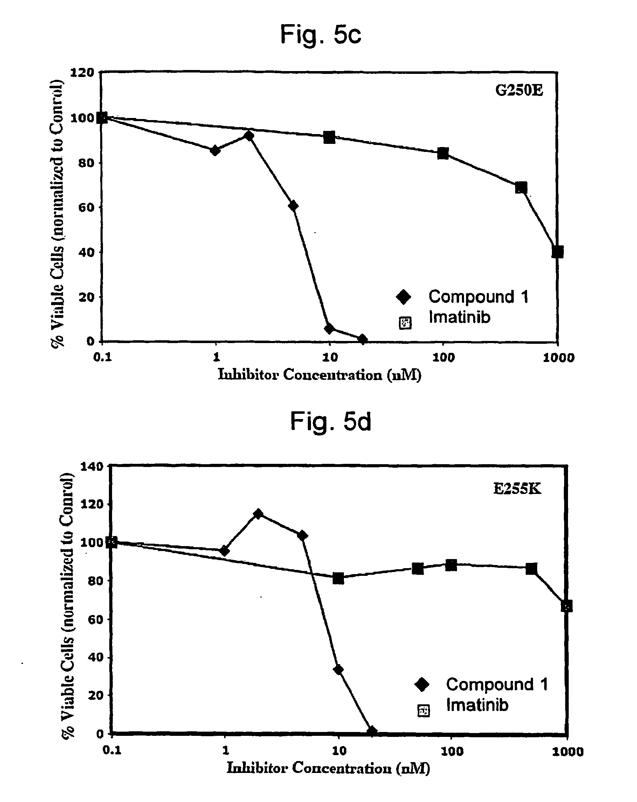

The result of four selected cell lines expressing *BCR-ABL* mutants T315I, E255K, Y253H and G250E are shown in Figures 5a, 5b, 5c and 5d, respectively.

### Example 5: Activity of Compound 2 ((E)-2-(5-((2,4,6-trimethoxystyrylsulfonyl) methyl)-2-methoxyphenylamino) acetic acid) Against Imatinib-resistant Tumor Cell Lines.

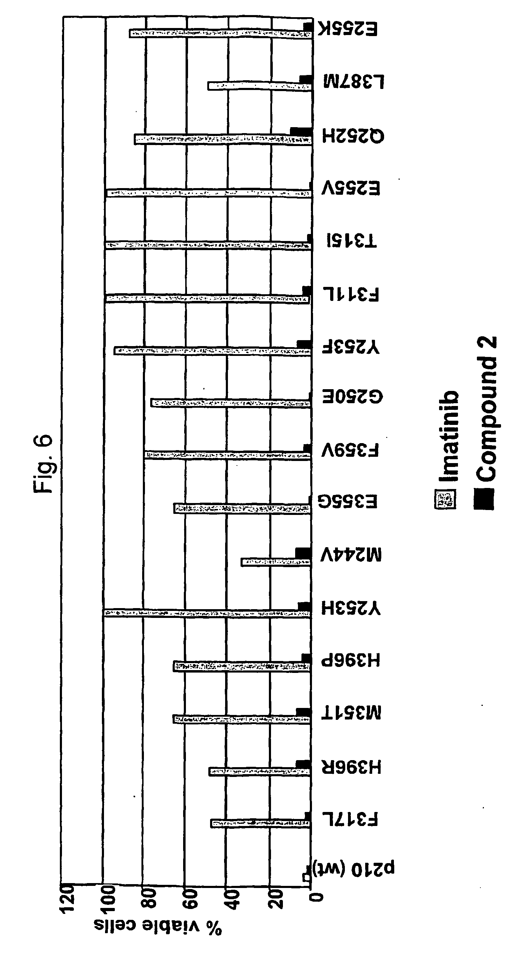

The cell lines listed in Table 2 were cultured in the presence of 250 nM Compound 2 or imatinib. The results are set forth in Figure 6. All of the cell lines were sensitive to the growth-inhibitory activity of Compound 2.

### Example 6: Treatment of Imatinib-resistant CML in Chronic Phase.

An adult patient diagnosed with chronic myelogenous leukemia is found to be imatinib-resistant. Sequencing of the *bcr-abl* gene expressed by leukemic cells confirms the presence of a mutation in the *bcr-abl* kinase domain. The patient's disease is in the chronic phase (fewer than 10% blasts, or 20% blasts and promyelocytes combined, in blood or bone marrow samples). The patient is treated with Compound 1 using the following dosing regimen: 200 milligrams of Compound 1 is administered orally once daily. Treatment is continued as long as there is no evidence of progressive disease or unacceptable toxicity. Upon appearance of disease progression to the accelerated phase (bone marrow or blood samples have more than 10% blasts (or 20% blasts and promyelocytes combined) but fewer than 30% blasts and promyelocytes), the daily dose of Compound 1 is increased to 300 milligrams. If necessary, additional dose increases are made, if no unacceptable toxicity occurs, to achieve the desired therapeutic result.

### Example 7: Treatment of Imatinib-resistant CML in Blast Phase.

An adult patient diagnosed with chronic myelogenous leukemia is found to be imatinib-resistant. Sequencing of the *bcr-abl* gene expressed by leukemic cells confirms the presence of a mutation in the *ber-abl* kinase domain. The patient's disease is in the blast phase. The blast phase (also known as acute phase or blast crisis) is characterized by bone marrow and/or blood samples have more than 30% blasts and promyelocytes. The patient is treated with Compound 1 using the following dosing regimen: 300 milligrams of Compound 1 is administered orally once daily. Treatment is continued as long as there is no evidence of progressive disease or unacceptable toxicity. Upon appearance of disease progression, the dose is increased to 400 milligrams, administered in a single dose or optionally, in two 200 milligrams doses daily. If necessary, additional dose increases are made, ifno unacceptable toxicity occurs, to achieve the desired therapeutic result.

### Example 8: Treatment of CML Following Initial Treatment with Imatinib.

An adult patient diagnosed with chronic myelogenous leukemia is initially treated with imatinib. Over time, the patient's disease becomes imatinib-resistant. Sequencing of the *bcr-abl* gene expressed by leukemic cells confirms the presence of a mutation in the *bcr-abl* kinase domain. The patient's treatment regimen is changed to a combination regimen as follows: Imatinib is increased to the maximum dose tolerated by patient (800 milligrams) and is administered twice daily as 400 milligram doses. A 300 milligram dose of Compound 1 is also administered orally once daily. Treatment is continued as long as there is no evidence of progressive disease or unacceptable toxicity. Upon appearance of disease progression, the amount of Compound 1 is increased as necessary to achieve the desired therapeutic result.

### Example 9: First-line Treatment of CML with Two Drugs.

An adult patient is newly diagnosed with chronic myelogenous leukemia. Sensitivity to imatinib is unknown. As a first line therapy, the patient is administered a combination of imatinib and Compound 1 using the following dosing regimen: A once daily oral dose comprising 400 milligrams of imatinib and 100 milligrams of Compound 1 is administered. Treatment is continued as long as there is no evidence of progressive disease or unacceptable toxicity. Upon appearance of disease progression, the dose of imatinib is increased to 600 milligrams and the dose of Compound 1 is increased to 200 milligrams. If necessary, additional dose increases, if no unacceptable toxicity occurs, in either or both imatinib (up to 800 milligrams) and Compound 1 are made.

### Example 10: Activity of Compound 3 ((E)-2-(5-((2,4,6-trimethoxystyrylsulfonyl)methyl)-2-methoxyphenylamino)-2-methylpropanoic acid sodium salt) Against Imatinib-resistant Tumor Cell Lines.

The cell lines listed in Table 3 were cultured in the presence of various concentrations of Compound 3 ((*E*)-2-(5-((2,4,6-trimethoxystyrylsulfonyl)methyl)-2-methoxyphenylamino)-2-methylpropanoic acid sodium salt). The wild type comprises 32Dc13.*BCR-ABL* cells that ectopically expressed the wild-type p210*^{BCR-ABL}* oncoprotein. The total number of viable cells was determined by trypan blue exclusion at 72 hours post-treatment. The percent viable cells compared to vehicle-treated controls was determined. The sensitivity of the cell lines, expressed in terms of GI₅₀, is listed in Table 3. All of the cell lines were sensitive to the growth-inhibitory activity of Compound 3.

**Table 3: Compound 3-induced Growth Inhibition of Cells.**

| **Mutation** | **GI₅₀ (µM)** |
|---|---|
| Wild-type | 0.04 |
| T315I | 0.06 |
| G250E | 0.025 |
| M35IT | 0.06 |
| F317L | 0.06 |
| H396P | 0.05 |
| F311L | 0.07 |
| Y253F | 0.04 |
| L387M | 0.05 |
| Q252H | 0.04 |
| E255K | 0.05 |
| Y253F | 0.07 |
| Y253H | 0.03 |
| E355G | 0.05 |
| H396R | 0.06 |
| F359V | 0.07 |
| E255V | 0.05 |

### Example 11: Activity of Compound 4 ((E)-1-(2-(4-Bromobenzylsulfonyl)vinyl)-2,4-difluorobenzene) Against Imatinib-resistant Tumor Cell Lines.

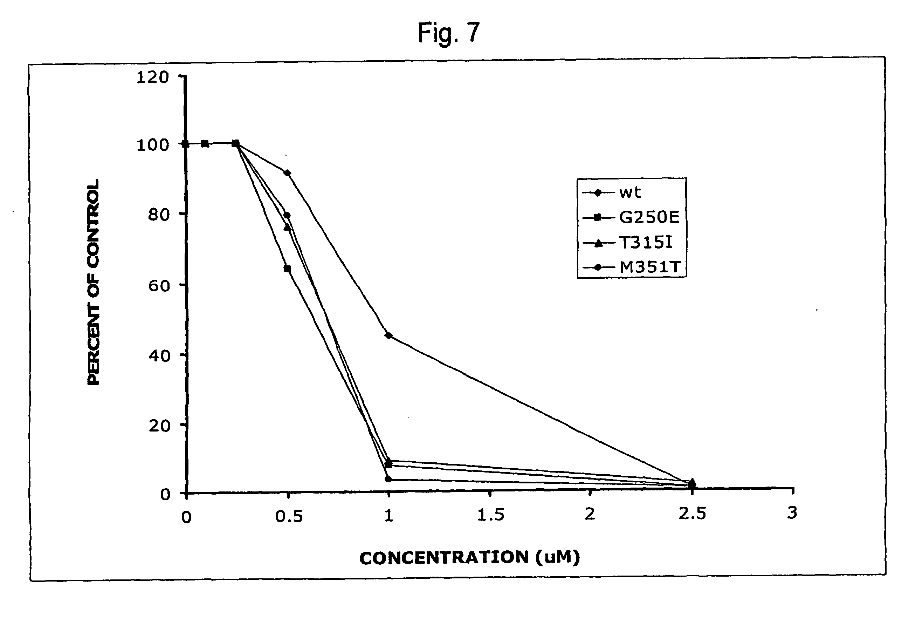

Cells expressing the wild-type p210*^{BCR-ABL}* oncoprotein (32Dcl3.*BCR-ABL*) or the *BCR-ABL* mutations G250E, T315I or M351T, were incubated with varying concentrations of Compound 4 ((*E*)-1-(2-(4-bromobenzylsulfonyl)vinyl)-2,4-difluorobenzene). The total number of viable cells was determined by trypan blue exclusion at 72 hours post-treatment. The percent viable cells compared to vehicle-treated controls was determined. The results are set forth in Fig. 7. All of the cell lines were sensitive to the growth-inhibitory activity of Compound 4.

One skilled in the art will readily appreciate that the present invention is well adapted to carry out the objects and obtain the ends and advantages mentioned, as well as those inherent therein. The present invention may be embodied in other specific forms without departing from the essential attributes thereof.

## Claims

1. Use of a compound for the manufacture of a medicament for treating a kinase-dependent proliferative disorder in an individual, which disorder is resistant to treatment with an ATP-competitive inhibitor of a kinase selected from the group consisting of BCR-ABL, KIT, epidermal growth factor receptor, PDGFRα, PDGFRβ, SRC and P38, which resistance results from mutation of said kinase, wherein said compound is a compound according to Formula I: 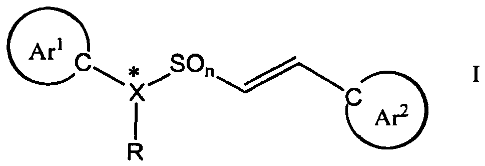 wherein:
Ar¹ and Ar² are independently selected from substituted and unsubstituted aryl and substituted and unsubstituted heteroaryl;
X is N or CH;
n is 1 or 2;
R is -H or -(C₁-C₈)hydrocarbyl; and
* indicates that, when X is CH, and R is other than -H, the conformation of the substituents on the carbon atom of X is *R*-, *S*- or any mixture of *R*- and *S*-; or a pharmaceutically acceptable salt thereof;
provided that when X is N, then n is 2.

2. The use according to claim 1, wherein substituents on substituted aryl and substituted heteroaryl Ar¹ are independently selected from the group consisting of halogen, -(C₁-C₈)hydrocarbyl, -C(=O)R², -NR²₂, -NHC(=O)R³, -NHSO₂R³, -NH(C₂-C₆)alkylene-C(=O)R⁶, -NHCR²R⁴C(=O)R⁶, -C(=O)OR²,
-C(=O)NR²₂, -NO₂, -CN, -OR², -OC(=O)R³, -OSO₂R³,
-O(C₂-C₆)alkylene-C(=O)R⁶, -OCR²R⁴C(=O)R⁶, -P(=O)(OR²)₂, -OP(=O)(OR²)₂, -O(C₂-C₆ alkylene)N(C₁-C₃)alkyl)₂, -NHC(=NH)NHR²,
-(C₁-C₆)haloakyl, -O(C₁-C₆)haloalkyl; -SO₂NH₂; and -N=CH-R⁷; and
substituents on substituted aryl and substituted heteroaryl Ar² are independently selected from the group consisting of -(C₁-C₈)hydrocarbyl, -C(=O)R², halogen, -NO₂, -CN, -OR², -C(=O)OR², -NR²₂, -(C₁-C₆)haloalkyl; -SO₂NH₂; and -O(C₁-C₆)haloalkyl; wherein:
each R² is independently selected from the group consisting of -H and -(C₁-C₈)hydrocarbyl;
each R³ is independently selected from the group consisting of -(C₁-C₈)hydrocarbyl, -O(C₁-C₈)hydrocarbyl, substituted and unsubstituted aryl, substituted and unsubstituted heterocyclyl(C₁-C₃)alkyl, substituted and unsubstituted heteroaryl(C₁-C₃)alkyl, -(C₂-C₁₀)heteroalkyl, -(C₁-C₆)haloalkyl, -CR²R⁴NHR⁵, -NR²₂, -(C₁-C₃)alkyleneNH₂, -(C₁-C₃)alkylene-N((C₁-C₃)alkyl)₂, -(C₁-C₃)perfluoroalkylene-N((C₁-C₃)alkyl)₂, -(C₁-C₃)alkylene-N⁺((C₁-C₃)alkyl)₃, -(C₁-C₃)alkylene-N⁺(CH₂CH₂OH)₃, -(C₁-C₃)alkylene-OR², -(C₁-C₄)alkylene-CO₂R², -(C₁-C₄)alkylene-C(=O)halogen, halo(C₁-C₃)alkyl-, -(C₁-C₃)alkylene-C(=O)(C₁-C₃)alkyl, and -(C₁-C₄)perfluoroalkylene-CO₂R²;
each R⁴ is independently selected from the group consisting of -H, -(C₁-C₆)alkyl, -(CH₂)₃-NH-C(NH₂)(=NH), -CH₂C(=O)NH₂, -CH₂CO₂R², -CH₂SH, -(CH₂)₂C(=O)-NH₂, -(CH₂)₂CO₂R², -CH₂-(2-imidazolyl), -(CH₂)₄-NH₂, -(CH₂)₂-S-CH₃, phenyl, -CH₂-phenyl, -CH₂-OH, -CH(OH)-CH₃, -CH₂-(3-indolyl), and -CH₂-(4-hydroxyphenyl);
each R⁵ is independently selected from the group consisting of -H, -C(=O)(C₁-C₇)hydrocarbyl and a carboxy terminally-linked peptidyl residue containing from 1 to 3 amino acids in which the terminal amino group of the peptidyl residue is present as a functional group selected from the group consisting of -NH₂, -NHC(=O)(C₁-C₆)alkyl, -NH(C₁-C₆)alkyl,
-N(C₁-C₆ alkyl)₂ and -NHC(=O)O(C₁-C₇)hydrocarbyl;
each R⁶ is independently selected from the group consisting of -OR², -NR²₂, and an amino terminally-linked peptidyl residue containing from 1 to 3 amino acids in which the terminal carboxyl group of the peptidyl residue is present as a functional group selected from the group consisting of -CO₂R² and -C(=O)NR²₂; and
each R⁷ is independently selected from the group consisting of substituted and unsubstituted aryl and substituted and unsubstituted heteroaryl;
provided that the highest number of substituents on Ar¹ and Ar² is equal to the number of substitutable hydrogen atoms in the ring to which the substituents are attached.

3. The use according to claim 2 wherein the compound according to Formula 1 is selected from the group consisting of: (*E*)-2-(5-((2,4,6-trimethoxystyrylsulfonyl)methyl)-2-methoxyphenylamino)acetic acid; (*E*)-(racemic)-2-(5-((2,4,6-trimethoxystyrylsulfonyl)methyl)-2-methoxyphenylamino)propanoic acid; (*R*)-(*E*)-2-(5-((2,4,6-trimethoxystyrylsulfonyl)methyl)-2-methoxyphenylamino)propanoic acid; (*S*)-(*E*)-2-(5-((2,4,6-trimethoxystyrylsulfonyl)methyl)-2-methoxyphenylamino)propanoic acid; (*E*)-2-(5-((2,4,6-trimethoxystyrylsulfinyl)methyl)-2-methoxyphenylamino)acetic acid; (*E*)-2-(5-((2,4,6-trimethoxystyryisulfinyl)methyl)-2-methoxyphenylamino)propanoic acid; (*E*)-2,4,6-trimethoxystyryl-*N*-(3-carboxymethylamino-4-methoxy-phenyl)sulfonamide; (*S*) (*E*)-2-(5-((2,4,6-trimethoxystyrylsulfonyl)methyl)-2-methoxyphenylamino)-2-phenylacetic acid; (*R*)-(*E*)-2-(5-((2,4,6-trimethoxystyrylsulfonyl)methyl)-2-methoxyphenylamino)-2-phenylacetic acid; (racemic)-(*E*)-2-(5-((2,4,6-trimethoxystyrylsulfonyl)methyl)-2-methoxyphenylamino)-2-phenylacetic acid; and pharmaceutically acceptable salts thereof.

4. The use according to claim 2 wherein the compound according to Formula I is selected from the group consisting of (*E*)-2-(5-((2,4,6-trimethoxystyrylsulfonyl)methyl)-2-methoxyphenylamino)-2-methylpropanoic acid; (*E*)-1-(2-(4-bromobenzylsulfonyl)vinyl)-2,4-difluorobenzene; and pharmaceutically acceptable salts thereof.

5. The use according to claim 2 wherein the compound according to Formula 1 comprises an isolated optical isomer, substantially free of the opposite enantiomer.

6. The use according to claim 5 wherein the isolated optical isomer has the (*R*)- absolute configuration at the atom designated by *, and is substantially free of the (*S*)-enantiomer.

7. The use according to claim 5 wherein the isolated optical isomer has the (*S*)- absolute configuration at the atom designated by *, and is substantially free of the (*R*)-enantiomer.

8. The use according to claim 1, wherein the kinase-dependent proliferative disorder is resistant to treatment with at least one compound selected from the group consisting of PD180970, BMS-354825, imatinib, SU5416, SU6668, SU11248, AP23464, gefitinib, erlotinib, PD153035, and SB203580.

9. The use according to claim 1, wherein the proliferative disorder is resistant to treatment with an ATP-competitive inhibitor of *BCR-ABL*.

10. The use according to claim 9, wherein the ATP-competitive inhibitor of *BCR-ABL* is imatinib.

11. The use according to claim 9, wherein the mutation comprises alteration of at least one amino acid residue within the *BCR-ABL* p-loop.

12. The use according to claim 11, wherein the mutation comprises an alteration of Tyr 253 or Glu255.

13. The use according to claim 9, wherein the mutation comprises alteration of at least one amino acid residue within the *BCR-ABL* activation loop.

14. The use according to claim 13, wherein the mutation comprises an alteration of His396.

15. The use according to claim 9, wherein the mutation comprises at least one mutation selected from the group consisting of F317L, H396R, M351T, H396P, Y253H, M244V, E355G, F359V, G250E, Y253F, F311L, T315I, E255V, Q252H, L387M, and E255K.

16. The use according to claim 1, wherein the proliferative disorder is selected from the group consisting of chronic myelogenous leukemia, acute lymphoblastic lymphoma, idiopathic pulmonary fibrosis, idiopathic hypereosinophilic syndrome, chronic myelomonocytic leukemia, malignant fibrous histiocytoma, prostate cancers, androgen dependent prostate cancers, dermatofibrosarcoma, endometrioid endometrial carcinoma, uterine papillary serous carcinoma, chordoma, glioma, malignant astrocytoma, glioblastoma, gastrointestinal stromal tumors, medulloblastoma, uterine leiomyosarcomas, and non-small-cell lung cancer.

17. The use according to claim 1, wherein the proliferative disorder is resistant to treatment with an ATP-competitive inhibitor of *KIT*.

18. The use according to claim 17, wherein the mutation is selected from the group consisting T670I, Y823D and D816V.

19. The use according to claim 1, wherein the proliferative disorder is resistant to treatment with an ATP-competitive inhibitor of epidermal growth factor receptor.

20. The use according to claim 19, wherein the mutation comprises the mutation T766M.

21. The use according to claim 1, wherein the proliferative disorder is resistant to treatment with an ATP-competitive inhibitor of PDGFRα.

22. The use according to claim 21, wherein the mutation comprises the mutation T674I.

23. The use according to claim 1, wherein the proliferative disorder is resistant to treatment with an ATP-competitive inhibitor of PDGFRβ.

24. The use according to claim 23 wherein the mutation comprises T681I.

25. Use of a compound for the manufacture of a medicament for preventing or delaying the development of resistance to therapy in an individual suffering from a kinase dependent proliferative disorder which includes administration of at least one ATP-competitive inhibitor of a kinase selected from the group consisting of BCR-ABL, KIT, epidermal growth factor receptor, PDGFRα, PDGFRβ, SRC and P38, said resistance resulting from mutation of said kinase, wherein said compound is a compound according to Formula I: 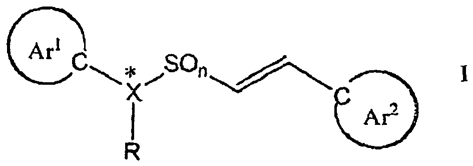 wherein:
Ar¹ and Ar² are independently selected from substituted and unsubstituted aryl and substituted and unsubstituted heteroaryl;
X is N or CH;
n is 0, 1 or 2;
R is -H or -(C₁-C₈)hydrocarbyl; and
* indicates that, when X is CH, and R is other than -H, the conformation of the substituents on the carbon atom of X is R-, S- or any mixture of R- and S-; or a pharmaceutically acceptable salt thereof;
provided that when X is N, then n is 2.

26. The use according to claim 25, wherein the at least one ATP-competitive kinase inhibitor is selected from the group consisting of PD 180970, BMS-354825, imatinib, SU5416, SU6668, SU11248, AP23464, gefitinib, erlotinib, PD153035, and SB203580.

## Patentansprüche

1. Eine Verwendung einer Verbindung für die Herstellung eines Medikaments zum Behandeln einer kinaseabhängigen proliferativen Störung in einem Individuum, wobei die Störung resistent ist gegenüber einer Behandlung mit einem ATP-kompetitiven Inhibitor einer Kinase, ausgewählt aus der Gruppe, bestehend aus BCR-ABL, KIT, epidermalem Wachstumsfaktorrezeptor, PDGFRα, PDGFRβ, SCR und P38, wobei die Resistenz aus einer Mutation der Kinase resultiert, wobei die Verbindung eine Verbindung gemäß Formel I ist: 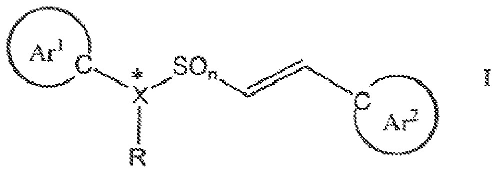 wobei:
Ar¹ und Ar² unabhängig aus substituiertem und nichtsubstituiertem Aryl und substituiertem und nichtsubstituiertem Heteroaryl ausgewählt sind;
X N oder CH ist;
n 1 oder 2 ist;
R -H oder -(C₁-C₈)Kohlenwasserstoffrest ist; und
* zeigt, dass, wenn X CH ist und R nicht gleich -H ist, die Konformation der Substituenten auf dem Kohlenstoffatom von X *R*-, *S*- oder eine beliebige Mischung von *R*- und *S*- ist; oder ein pharmazeutisch annehmbares Salz davon, unter der Bedingung, dass wenn X N ist, dann n 2 ist.

2. Verwendung gemäß Anspruch 1, wobei Substituenten auf substituiertem Aryl und substituiertem Heteroaryl Ar¹ unabhängig aus der Gruppe, bestehend aus Halogen, -(C₁- C₈)Kohlenwasserstoffrest, -C(=O)R², -NR²₂, -NHC(=O)R³, -NHSO₂R³, -NH(C₂-C₆)Alkylen-C(=O)R⁶, -NHCR²R⁴C(=O)R⁶, -C(=O)OR², -C(=O)NR²₂, -NO₂, -CN, -OR², -OC(=O)R³, -OSO₂R³,-O(C₂-C₆)Alkylen-C(=O)R⁶, -OCR²R⁴C(=O)R⁶, -P(=O)(OR²)₂, -OP)(=O)(OR²)₂, -O(C₂-C₆ Alkylen)N(C₁-C₃)alkyl)₂, -NHC(=NH)NHR², -(C₁-C₆)Halogenalkyl, -O(C₁-C₆)Halogenalkyl, -SO₂NH und -N=CH-R⁷, ausgewählt sind; und
Substituenten auf substituiertem Aryl und substituiertem Heteroaryl Ar² unabhängig aus der Gruppe, bestehend aus -(C₁-C₈)Kohlenwasserstoffrest, -C(=O)R², Halogen, -NO₂, -CN, -OR², -C(=O)OR², -NR²₂, -(C₁-C₆)Halogenalkyl; -SO₂NH₂ und -O(C₁-C₆)Halogenalkyl, ausgewählt sind; wobei:
jedes R² unabhängig aus der Gruppe, bestehend aus -H und -(C₁-C₈)Kohlenwasserstoffrest, ausgewählt ist;
jedes R³ unabhängig aus der Gruppe, bestehend aus -(C₁-C₈)Kohlenwasserstoffrest, -O(C₁-C₈)Kohlenwasserstoffrest, substituiertem und nichtsubstituiertem Aryl, substituiertem und nichtsubstituiertem Heterocyclyl(C₁-C₃)alkyl, substituiertem und nichtsubstituiertem Heteroaryl(C₁-C₃)alkyl, -(C₂-C₁₀)Heteroalkyl, -(C₁-C₆)Halogenalkyl, -CR²R⁴NHR⁵, -NR²₂, -(C₁-C₃)AlkylenNH₂, -(C₁-C₃)Alkylen-N((C₁-C₃)alkyl)₂, -(C₁-C₃)Perfluoralkylen-N((C₁-C₃)alkyl)₂, -(C₁-C₃)Alkylen-N⁺((C₁-C₃)alkyl)₃, -(C₁-C₃)Alkylen-N⁺(CH₂CH₂OH)₃, -(C₁-C₃)Alkylen-OR², -(C₁-C₄)Alkylen-CO₂R², -(C₁-C₄)AlkylenC(=O)halogen, Halogen(C₁-C₃)alkyl-, -(C₁-C₃)Alkylen-C(=O)(C₁-C₃)alkyl und -(C₁-C₄)Perfluoralkylen-CO₂R², ausgewählt ist; jedes R⁴ unabhängig aus der Gruppe, bestehend aus -H, -(C₁-C₆)Alkyl, -(CH₂)₃-NH-C(NH₂)(=NH), -CH₂C(=O)NH₂, -CH₂CO₂R², -CH₂SH, -(CH₂)₂C(=O)-NH₂, -(CH₂)₂CO₂R², -CH₂-(2-Imidazolyl), -(CH₂)₄-NH₂, -(CH₂)₂-S-CH₃, Phenyl, -CH₂-Phenyl, -CH₂-OH, CH(OH)-CH₃, -CH₂-(3-Indolyl) und -CH₂-(4-Hydroxyphenyl), ausgewählt ist;
jedes R⁵ unabhängig aus der Gruppe, bestehend aus -H, -C(=O)(C₁-C₇)Kohlenwasserstoffrest und einem carboxy-terminal verknüpften Peptidylrest, der von 1 bis 3 Aminosäuren enthält, in denen die terminale Aminogruppe des Peptidylrests vorhanden ist als eine funktionale Gruppe, ausgewählt aus der Gruppe, bestehend aus -NH₂, -NHC(=O)(C₁-C₆)Alkyl, -NH(C₁-C₆)Alkyl, -N(C₁-C₆ Alkyl)₂ und -NHC(=O)O(C₁-C₇)Kohlenwasserstoffrest, ausgewählt ist;
jedes R⁶ unabhängig aus der Gruppe, bestehend aus -OR², -NR²₂ und einem amino-terminal verknüpften Peptidylrest, der von 1 bis 3 Aminosäuren enthält, in denen die terminale Carboxylgruppe des Peptidylrests vorhanden ist als eine funktionale Gruppe, ausgewählt aus der Gruppe, bestehend aus -CO₂R² und -C(=O)NR²₂, ausgewählt ist; und
jedes R⁷ unabhängig aus der Gruppe, bestehend aus substituiertem und nichtsubstituiertem Aryl und substituiertem und nichtsubstituiertem Heteroaryl, ausgewählt ist;
unter der Bedingung, dass die höchste Anzahl an Substituenten auf Ar¹ und Ar² gleich der Anzahl an substituierbaren Wasserstoffatomen in dem Ring, an dem die Substituenten hängen.

3. Verwendung gemäß Anspruch 2, wobei die Verbindung gemäß Formel I aus der Gruppe, die aus Folgendem besteht, ausgewählt ist: (*E*)-2-(5-((2,4,6-Trimethoxystyrylsulfonyl)methyl)-2-methoxyphenylamino)essigsäure; (*E*)-(racemisch)-2-(5-((2,4,6-Trimethoxystyrylsulfonyl)methyl)-2-methoxyphenylamino)propansäure; *(R)*-*(E)*-2-(5-((2,4,6-Trimethoxystyrylsulfonyl)methyl)-2-methoxyphenylamino)propansäure; *(S)*-*(E)*-2-(5-((2,4,6-Trimethoxystyrylsulfonyl)methyl)-2-methoxyphenylamino)propansäure; (*E*)-2-(5-((2,4,6-Trimethoxystyrylsulfinyl)methyl)-2-methoxyphenylamino)essigsäure; (*E*)-2-(5-((2,4,6-Trimethoxystyrylsulfinyl)methyl)-2-methoxyphenylamino)propansäure; (*E*)-2,4,6-Trimethoxystyryl-*N*-(3-carboxymethylamino-4-methoxyphenyl)sulfonamid; *(S)* (*E*)-2-(5-((2,4,6-Trimethoxystyrylsulfonyl)methyl)-2-methoxyphenylamino)-2-phenylessigsäure; (*R*)-(*E*)-2-(5-((2,4,6-Trimethoxystyrylsulfonyl)methyl)-2-methoxyphenylamino)-2-phenylessigsäure; (racemisch)-(*E*)-2-(5-((2,4,6-Trimethoxystyrylsulfonyl)methyl)-2-methoxyphenylamino)-2-phenylessigsäure; und pharmazeutisch annehmbare Salze davon.

4. Verwendung gemäß Anspruch 2, wobei die Verbindung gemäß Formel I aus der Gruppe, bestehend aus (*E*)-2-(5-((2,4,6-Trimethoxystyrylsulfonyl)methyl)-2-methoxyphenylamino)-2-methylpropansäure, (*E*)-1-(2-(4-Brombenzylsulfonyl)vinyl)-2,4-difluorbenzen und pharmazeutisch annehmbaren Salzen davon, ausgewählt ist.

5. Verwendung gemäß Anspruch 2, wobei die Verbindung gemäß Formel I ein isoliertes optisches Isomer beinhaltet, das im Wesentlichen frei vom entgegengesetzten Enantiomer ist.

6. Verwendung gemäß Anspruch 5, wobei das isolierte optische Isomer die *(R)-*absolute Konfiguration am Atom, das mit * bezeichnet ist, aufweist und im Wesentlichen frei vom *(S)*-Enantiomer ist.

7. Verwendung gemäß Anspruch 5, wobei das isolierte optische Isomer die *(S)-*absolute Konfiguration am Atom, das mit * bezeichnet ist, aufweist und im Wesentlichen frei vom *(R)*-Enantiomer ist.

8. Verwendung gemäß Anspruch 1, wobei die kinaseabhängige proliferative Störung resistent ist gegenüber einer Behandlung mit mindestens einer Verbindung, ausgewählt aus der Gruppe, bestehend aus PD180970, BMS-354825, Imatinib, SU5416, SU6668, SU11248, AP23464, Gefitinib, Erlotinib, PD153035 und SB203580.

9. Verwendung gemäß Anspruch 1, wobei die proliferative Störung resistent ist gegenüber einer Behandlung mit einem ATP-kompetitiven Inhibitor von *BCR-ABL*.

10. Verwendung gemäß Anspruch 9, wobei der ATP-kompetitive Inhibitor von *BCR-ABL* Imatinib ist.

11. Verwendung gemäß Anspruch 9, wobei die Mutation eine Veränderung von mindestens einem Aminosäurerest innerhalb der *BCR-AB*L-p-Schleife beinhaltet.

12. Verwendung gemäß Anspruch 11, wobei die Mutation eine Veränderung von Tyr 253 oder Glu255 beinhaltet.

13. Verwendung gemäß Anspruch 9, wobei die Mutation eine Veränderung von mindestens einem Aminosäurerest innerhalb der *BCR-ABL*-Aktivierungschleife beinhaltet.

14. Verwendung gemäß Anspruch 13, wobei die Mutation eine Veränderung von His396 beinhaltet.

15. Verwendung gemäß Anspruch 9, wobei die Mutation mindestens eine Mutation, ausgewählt aus der Gruppe, bestehend aus F317L, H396R, M351 T, H396P, Y253H, M244V, E355G, F359V, G250E, Y253F, F311 L, T315I, E255V, Q252H, L387M und E255K, beinhaltet.

16. Verwendung gemäß Anspruch 1, wobei die proliferative Störung aus der Gruppe, bestehend aus chronischer myeloischer Leukämie, akutem lymphoblastischem Lymphom, idiopathischer Lungenfibrose, idiopathischem Hypereosinophilie-Syndrom, chronischer myelomonozytärer Leukämie, malignem fibrösem Histiozytom, Prostatakrebsen, androgenabhängigen Prostatakrebsen, Dermatofibrosarkom, endometrioidem Endometriumkarzinom, papillärem serösem Uteruskarzinom, Chordom, Gliom, malignem Astrozytom, Glioblastom, gastrointestinalen Stromatumoren, Medulloblastom, uterinen Leiomyosarkomen und nicht-kleinzelligem Lungenkrebs, ausgewählt ist.

17. Verwendung gemäß Anspruch 1, wobei die proliferative Störung resistent ist gegenüber einer Behandlung mit einem ATP-kompetitiven Inhibitor von *KIT*.

18. Verwendung gemäß Anspruch 17, wobei die Mutation aus der Gruppe, bestehend aus T670I, Y823D and D816V, ausgewählt ist.

19. Verwendung gemäß Anspruch 1, wobei die proliferative Störung resistent ist gegenüber einer Behandlung mit einem ATP-kompetitiven Inhibitor von epidermalem Wachstumsfaktorrezeptor.

20. Verwendung gemäß Anspruch 19, wobei die Mutation die Mutation T766M beinhaltet.

21. Verwendung gemäß Anspruch 1, wobei die proliferative Störung resistent ist gegenüber einer Behandlung mit einem ATP-kompetitiven Inhibitor von PDGFRα.

22. Verwendung gemäß Anspruch 21, wobei die Mutation die Mutation T674I beinhaltet.

23. Verwendung gemäß Anspruch 1, wobei die proliferative Störung resistent ist gegenüber einer Behandlung mit einem ATP-kompetitiven Inhibitor von PDGFRβ.

24. Verwendung gemäß Anspruch 23, wobei die Mutation T681I beinhaltet.

25. Eine Verwendung einer Verbindung für die Herstellung eines Medikaments zum Verhindern oder Verlangsamen der Entwicklung einer Resistenz gegenüber einer Therapie in einem Individuum, das an einer kinaseabhängigen proliferativen Störung leidet, die die Verabreichung von mindestens einem ATP-kompetitiven Inhibitor einer Kinase, ausgewählt aus der Gruppe, bestehend aus BCR-ABL, KIT, epidermalem Wachstumsfaktorrezeptor, PDGFRα, PDGFRβ, SCR und P38, einschließt, wobei die Resistenz aus einer Mutation der Kinase resultiert, wobei die Verbindung eine Verbindung gemäß Formel I ist: 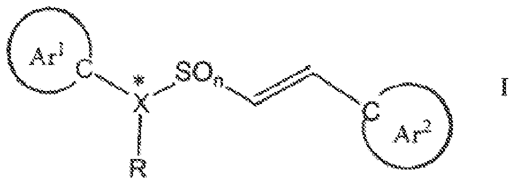 wobei:
Ar¹ und Ar² unabhängig ausgewählt sind aus substituiertem und nichtsubstituiertem Aryl und substituiertem und nichtsubstituiertem Heteroaryl;
X N oder CH ist;
n 0, 1 oder 2 ist;
R -H oder -(C₁-C₈)Kohlenwasserstoffrest ist; und
* zeigt, dass, wenn X CH ist und R nicht gleich -H ist, die Konformation der Substituenten auf dem Kohlenstoffatom von X *R*-, *S*- oder eine beliebige Mischung von *R*- und *S*- ist; oder ein pharmazeutisch annehmbares Salz davon,
unter der Bedingung, dass wenn X N ist, dann n 2 ist.

26. Verwendung gemäß Anspruch 25, wobei der mindestens eine ATP-kompetitive Kinase-Inhibitor aus der Gruppe, bestehend aus PD180970, BMS-354825, Imatinib, SU5416, SU6668, SU11248, AP23464, Gefitinib, Erlotinib, PD153035 und SB203580, ausgewählt ist.

## Revendications

1. Utilisation d'un composé pour la fabrication d'un médicament pour traiter un trouble prolifératif kinase-dépendant chez un individu, lequel trouble est résistant à un traitement avec un inhibiteur compétitif de l'ATP d'une kinase sélectionnée dans le groupe consistant en BCR-ABL, KIT, récepteur de facteur de croissance épidermique, PDGFRα, PDGFRβ, SRC et P38, laquelle résistance résulte d'une mutation de ladite kinase, ledit composé étant un composé selon la Formule I : 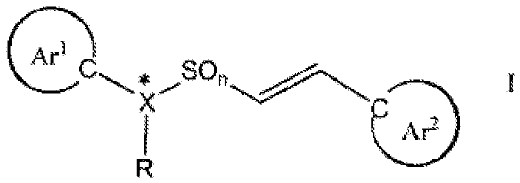 dans laquelle :
Ar¹ et Ar² sont indépendamment sélectionnés parmi un aryle substitué et non substitué et un hétéroaryle substitué et non substitué ;
X est N ou CH ;
n est 1 ou 2 ;
R est -H ou -(C₁-C₈)hydrocarbyle ; et
* indique que, lorsque X est CH, et R est autre que -H, la conformation des substituants sur l'atome de carbone de X est *R*-, *S*- ou tout mélange de *R*- et *S*- ; ou un sel acceptable d'un point de vue pharmaceutique de ceux-ci ;
à condition que lorsque X est N, alors n est 2.

2. L'utilisation selon la revendication 1, dans laquelle des substituants sur l'aryle substitué et l'hétéroaryle substitué Ar¹ sont indépendamment sélectionnés dans le groupe consistant en halogène, -(C₁-C₈)hydrocarbyl, -C(=O)R², -NR²₂, -NHC(=O)_{R}³, -NHSO₂R³, -NH(C₂-C₆)alkylène-C(=O)R⁶, -NHCR²R⁴C(=O)R⁶, -C(=O)OR², -C(=O)NR²₂, -NO₂, -CN, -OR², -OC(=O)R³, -OSO₂R³, -O(C₂-C₆)alkylène-C(=O)R⁶, -OCR²R⁴C(=O)R⁶, -P(=O)(OR²)₂, -OP(=O)(OR²)₂, -O(C₂-C₆alkylène)N(C₁-C₃)alkyl)₂, -NHC(=NH)NHR², -(C₁-C₆)haloalkyl, -O(C₁-C₆)haloalkyl ; -SO₂NH₂ ; et -N=CH-R⁷ ; et des substituants sur l'aryle substitué et l'hétéroaryl substitué Ar² sont indépendamment sélectionnés dans le groupe consistant en -(C₁-C₈)hydrocarbyl, -C(=O)R², halogène, -NO₂, -CN, -OR², -C(=O)OR², -NR²₂, -(C₁-C₆)haloalkyl ; -SO₂NH₂ ; et -O(C₁-C₆)haloalkyl ; dans laquelle :
chaque R² est indépendamment sélectionné dans le groupe consistant en -H et -(C₁-C₈)hydrocarbyl ;
chaque R³ est indépendamment sélectionné dans le groupe consistant en -(C₁-C₈)hydrocarbyl, -O(C₁-C₈)hydrocarbyl, un aryle substitué et non substitué, un hétérocyclyl(C₁-C₃)alkyl substitué et non substitué, un hétéroaryle(C₁-C₃)alkyl substitué et non substitué, -(C₂-C₁₀)hétéroalkyl, -(C₁-C₆)haloalkyl, -CR²R⁴NHR⁵, -NR²₂, -(C₁-C₃)alkylèneNH₂, -(C₁-C₃)alkylène-N((C₁-C₃)alkyl)₂, -(C₁-C₃)perfluoroalkylène-N((C₁-C₃)alkyl)₂, -(C₁-C₃)alkylène-N⁺((C₁-C₃)alkyl)₃, -(C₁-C₃)alkylène-N⁺(CH₂CH₂OH)₃, -(C₁-C₃)alkylène-OR², -(C₁-C₄)alkylène-CO₂R², -(C₁-C₄)alkylène-C(=O)halogène, halo(C₁-C₃)alkyl-, -(C₁-C₃)alkylène-C(=O)(C₁-C₃)alkyl, et -(C₁-C₄)perfluoroalkylène-CO₂R² ;
chaque R⁴ est indépendamment sélectionné dans le groupe consistant en -H, -(C₁-C₆)alkyl, -(CH₂)₃-NH-C(NH₂)(=NH), -CH₂C(=O)NH₂, -CH₂CO₂R², -CH₂SH, -(CH₂)₂C(=O)-NH₂, -(CH₂)₂CO₂R², -CH₂-(2-imidazolyl), -(CH₂)₄-NH₂, -(CH₂)₂-S-CH₃, phényl, -CH₂-phényl, -CH₂-OH, -CH(OH)-CH₃, -CH₂-(3-indolyl), et -CH₂-(4-hydroxyphényl) ;
chaque R⁵ est indépendamment sélectionné dans le groupe consistant en -H, -C(=O)(C₁-C₇)hydrocarbyle et un résidu peptidyl à liaison terminale carboxy contenant de 1 à 3 acides aminés dans lesquels le groupe amino terminal du résidu peptidyl est présent comme groupe fonctionnel sélectionné dans le groupe consistant en -NH₂, -NHC(=O)(C₁-C₆)alkyl, -NH(C₁-C₆)alkyl, -N(C₁-C₆alkyl)₂ et -NHC(=O)O(C₁-C₇)hydrocarbyl ;
chaque R⁶ est indépendamment sélectionné dans le groupe consistant en -OR², -NR²₂, et un résidu peptidyl à liaison terminale amino contenant de 1 à 3 acides aminés dans lesquels le groupe carboxyl terminal du résidu peptidyl est présent comme groupe fonctionnel sélectionné dans le groupe consistant en -CO₂R² et -C(=O)NR²₂ ; et
chaque R⁷ est indépendamment sélectionné dans le groupe consistant en aryle substitué et non substitué et hétéroaryle substitué et non substitué ;
à condition que le nombre le plus élevé de substituants sur Ar¹ et Ar² soit égal au nombre d'atomes d'hydrogène substituables dans l'anneau auquel les substituants sont attachés.

3. L'utilisation selon la revendication 2 dans laquelle le composé selon la Formule I est sélectionné dans le groupe consistant en : (*E*)-2-(5-((2,4,6-triméthoxystyrylsulfonyl)méthyl)-2-méthoxyphénylamino)acide acétique ; (*E*)-(racémic)-2-(5-((2,4,6-triméthoxystyrylsulfonyl)méthyl)-2-méthoxyphénylamino)acide propanoïque ; (*R*)-(*E*)-2-(5-((2,4,6-triméthoxystyrylsulfonyl)méthyl)-2-méthoxyphénylamino)acide propanoïque ; (*S*)-(*E*)-2-(5-((2,4,6-triméthoxystyrylsulfonyl)méthyl)-2-méthoxyphénylamino)acide propanoïque ; (*E*)-2-(5-((2,4,6-triméthoxystyrylsulfinyl)méthyl)-2-méthoxyphénylamino)acide acétique ; (*E*)-2-(5-((2,4,6-triméthoxystyrylsulfinyl)méthyl)-2-méthoxyphénylamino)acide propanoïque ; (*E*)-2,4,6-triméthoxystyryl-*N*-(3-carboxyméthylamino-4-méthoxy-phényl)sulfonamide ; (*S*) (*E*)-2-(5-((2,4,6-triméthoxystyrylsulfonyl)méthyl)-2-méthoxyphénylamino)-2-acide phénylacétique ; (*R*)-(*E*)-2-(5-((2,4,6-triméthoxystyrylsulfonyl)méthyl)-2-méthoxyphénylamino)-2-acide phénylacétique ; (racémic)-(*E*)-2-(5-((2,4,6-triméthoxystyrylsulfonyl)méthyl)-2-méthoxyphénylamino)-2-acide phénylacétique ; et des sels acceptables d'un point de vue pharmaceutique de ceux-ci.

4. L'utilisation selon la revendication 2 dans laquelle le composé selon la Formule I est sélectionné dans le groupe consistant en (*E*)-2-(5-((2,4,6-triméthoxystyrylsulfonyl)méthyl)-2-méthoxyphénylamino)-2-acide méthylpropanoïque ; (*E*)-1-(2-(4-bromobenzylsulfonyl)vinyl)-2,4-difluorobenzène ; et des sels acceptables d'un point de vue pharmaceutique de ceux-ci.

5. L'utilisation selon la revendication 2 dans laquelle le composé selon la Formule I comprend un isomère optique isolé, substantiellement dépourvu d'énantiomère opposé.

6. L'utilisation selon la revendication 5 dans laquelle l'isomère optique isolé présente la configuration (*R*)-absolue au niveau de l'atome désigné par *, et est substantiellement dépourvu de (*S*)-énantiomère.

7. L'utilisation selon la revendication 5 dans laquelle l'isomère optique isolé présente la configuration (*S*)-absolue au niveau de l'atome désigné par *, et est substantiellement dépourvu de (*R*)-énantiomère.

8. L'utilisation selon la revendication 1, dans laquelle le trouble prolifératif kinase-dépendant est résistant au traitement avec au moins un composé sélectionné dans le groupe consistant en PD180970, BMS-354825, imatinib, SU5416, SU6668, SU11248, AP23464, gefitinib, erlotinib, PD153035, et SB203580.

9. L'utilisation selon la revendication 1, dans laquelle le trouble prolifératif est résistant au traitement avec un inhibiteur compétitif de l'ATP de *BCR-ABL.*

10. L'utilisation selon la revendication 9, dans laquelle l'inhibiteur compétitif de l'ATP de *BCR-ABL* est l'imatinib.

11. L'utilisation selon la revendication 9, dans laquelle la mutation comprend la modification d'au moins un résidu d'acide aminé dans la boucle P de *BCR-ABL.*

12. L'utilisation selon la revendication 11, dans laquelle la mutation comprend une modification de Tyr 253 ou Glu255.

13. L'utilisation selon la revendication 9, dans laquelle la mutation comprend la modification d'au moins un résidu d'acide aminé dans la boucle d'activation de *BCR-ABL.*

14. L'utilisation selon la revendication 13, dans laquelle la mutation comprend une modification de His396.

15. L'utilisation selon la revendication 9, dans laquelle la mutation comprend au moins une mutation sélectionnée dans le groupe consistant en F317L, H396R, M351T, H396P, Y253H, M244V, E355G, F359V, G250E, Y253F, F311 L, T315I, E255V, Q252H, L387M, et E255K.

16. L'utilisation selon la revendication 1, dans laquelle le trouble prolifératif est sélectionné dans le groupe consistant en leucémie myéloïde chronique, lymphome lymphoblastique aigu, fibrose pulmonaire idiopathique, syndrome hyperéosinophilique idiopathique, leucémie myélomonocitique chronique, histiocytofibrome malin, cancers de la prostate, cancers de la prostate androgéno-dépendants, dermatofibrosarcome, carcinome de l'endomètre endométrioïde, carcinome séreux papillaire utérin, chordome, gliome, astrocytome malin, glioblastome, tumeurs du stroma gastro-intestinale, médulloblastome, léiomyosarcomes utérins, et cancer du poumon non à petites cellules.

17. L'utilisation selon la revendication 1, dans laquelle le trouble prolifératif est résistant au traitement avec un inhibiteur compétitif de l'ATP de *KIT.*

18. L'utilisation selon la revendication 17, dans laquelle la mutation est sélectionnée dans le groupe consistant en T670I, Y823D et D816V.

19. L'utilisation selon la revendication 1, dans laquelle le trouble prolifératif est résistant au traitement avec un inhibiteur compétitif de l'ATP de récepteur de facteur de croissance épidermique.

20. L'utilisation selon la revendication 19, dans laquelle la mutation comprend la mutation T766M.

21. L'utilisation selon la revendication 1, dans laquelle le trouble prolifératif est résistant au traitement avec un inhibiteur compétitif de l'ATP de PDGFRα.

22. L'utilisation selon la revendication 21, dans laquelle la mutation comprend la mutation T674I.

23. L'utilisation selon la revendication 1, dans laquelle le trouble prolifératif est résistant au traitement avec un inhibiteur compétitif de l'ATP de PDGFRβ.

24. L'utilisation selon la revendication 23 dans laquelle la mutation comprend T681I.

25. Utilisation d'un composé pour la fabrication d'un médicament pour empêcher ou retarder le développement d'une résistance à une thérapie chez un individu souffrant d'un trouble prolifératif kinase-dépendant incluant l'administration d'au moins un inhibiteur compétitif de l'ATP d'une kinase sélectionnée dans le groupe consistant en BCR-ABL, KIT, récepteur de facteur de croissance épidermique, PDGFRα, PDGFRβ, SRC et P38, ladite résistance résultant d'une mutation de ladite kinase, ledit composé étant un composé selon la Formule I : 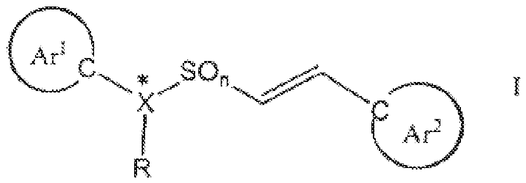 dans laquelle :
Ar¹ et Ar² sont indépendamment sélectionnés parmi un aryle substitué et non substitué et un hétéroaryle substitué et non substitué ;
X est N ou CH ;
n est 0, 1 ou 2 ;
R est -H ou -(C₁-C₈)hydrocarbyle ; et
* indique que, lorsque X est CH, et R est autre que -H, la conformation des substituants sur l'atome de carbone de X est *R*-, *S*- ou tout mélange de *R*- et *S*- ;
ou un sel acceptable d'un point de vue pharmaceutique de ceux-ci ;
à condition que lorsque X est N, alors n est 2.

26. L'utilisation selon la revendication 25, dans laquelle cet au moins un inhibiteur de kinase compétitif de l'ATP est sélectionné dans le groupe consistant en PD180970, BMS-354825, imatinib, SU5416, SU6668, SU 11248, AP23464, gefitinib, erlotinib, PD153035, et SB203580.
